# EUROPEAN PATENT SPECIFICATION

(11) **EP 3 387 149 B1**
(45) Date of publication and mention of the grant of the patent: **29.01.2020**
(21) Application number: 16820188.7
(22) Date of filing: 09.12.2016
(51) Int. Cl.: C12Q 1/68, C12Q 1/70, C12Q 1/6844

(54) **ISOTHERMAL AMPLIFICATION FOR THE DETECTION OF INFLUENZA VIRUSES IN A SAMPLE.**
ISOTHERMISCHE AMPLIFIZIERUNG ZUR DETEKTION VON INFLUENZA VIRUS IN EINER PROBE.
AMPLIFICATION ISOTHERMALE POUR LA DETECTION DU VIRUS INFLUENZA DANS UN ECHANTILLON

(30) Priority: 11.12.2015 EP 15199650
(43) Date of publication of application: 17.10.2018
(73) Proprietor: Orion Diagnostica Oy, 02200 Espoo (FI)
(72) Inventor: BRUMMER, Mirko, 02200 Espoo (FI); EBOIGBODIN, Kevin, 02200 Espoo (FI); ELF, Sonja, 02200 Espoo (FI); FILEN, Sanna, 02200 Espoo (FI); OJALEHTO, Tuomas, 02200 Espoo (FI)
(74) Representative: J A Kemp LLP
(86) International application number: PCT/EP2016/080525
(87) International publication number: WO 2017/098023

(56) References cited:
- WO-A1-2015/185655
- WO-A1-2016/028312
- US-A1- 2006 257 860
- US-A1- 2014 024 014
- MARK J. HOSER ET AL: "Strand Invasion Based Amplification (SIBA ): A Novel Isothermal DNA Amplification Technology Demonstrating High Specificity and Sensitivity for a Single Molecule of Target Analyte", PLOS ONE, vol. 9, no. 11, 24 November 2014 (2014-11-24), page e112656, XP055270716, DOI: 10.1371/journal.pone.0112656 cited in the application
- KEVIN EBOIGBODIN ET AL: "Reverse transcription strand invasion based amplification (RT-SIBA): a method for rapid detection of influenza A and B", APPLIED MICROBIOLOGY AND BIOTECHNOLOGY, 11 April 2016 (2016-04-11), XP055270717, DE ISSN: 0175-7598, DOI: 10.1007/s00253-016-7491-y

## Description

### Field of the Invention

The invention relates to a method for detecting a target nucleic acid sequence of a ribonucleic acid (RNA) comprising strand-invasion based DNA amplification. The invention also relates to oligonucleotides, compositions and kits suitable for use in this method, and their use for detection of pathogens, particularly influenza..

### Background to the Invention

Nucleic acid amplification tests are becoming the method of choice for routine diagnosis of pathogens such as viruses within clinical laboratory settings. They offer superior sensitivity and specificity over serology, immunoassays and traditional viral culture-based detection methods [Mahony et al. 2011].

Real-time reverse transcription polymerase chain reaction (RT-PCR) assays have been developed for example for the diagnosis of influenza and remain the most common platform used. A limitation of RT-PCR is that it can be prone to false negative results due to sample derived inhibition [Drosten et al. 2002]. Hence the RNA extraction step can become a limiting factor in the performance of the method. RT-PCR also requires the use of heavy and sophisticated thermal cyclers and skilled personnel, which consequently limits its use for in field or point-of-care applications.

An isothermal DNA amplification process relying on an upstream primer, a downstream primer, and a strand invasion oligonucleotide is described in WO 2009/150467 A1 and Hoser et al. 2014. Detection of C. difficile bacterial DNA has been described in WO 2014/173963A1.

### Summary of the Invention

In a first aspect, the present invention provides a method for detecting a ribonucleic acid (RNA) comprising a target nucleic acid sequence from an Influenza virus in a sample, said method comprising contacting said sample with a reverse transcriptase, at least one upstream primer, at least one downstream primer and at least one strand invasion oligonucleotide under conditions promoting amplification of said target nucleic acid sequence,
wherein each said primer and said strand invasion oligonucleotide comprises a region complementary to said target nucleic acid sequence;
wherein said strand invasion oligonucleotide renders at least a portion of a duplex nucleic acid comprising said target nucleic acid sequence single-stranded, to allow the binding of said upstream primer and a downstream primer;
wherein:
(i) the amplified target nucleic acid sequence comprises SEQ ID NO: 1, and the upstream primer comprises SEQ ID NO: 2 or a variant thereof and the downstream primer comprises SEQ ID NO: 3 or a variant thereof and the strand invasion oligonucleotide comprises SEQ ID NO: 4 or a variant thereof;
(ii) the amplified target nucleic acid sequence comprises SEQ ID NO: 6, and the upstream primer comprises SEQ ID NO: 7 or a variant thereof and the downstream primer comprises SEQ ID NO: 8 or a variant thereof and the strand invasion oligonucleotide comprises SEQ ID NO: 9 or a variant thereof;
(iii) the amplified target nucleic acid sequence comprises SEQ ID NO: 11, and the upstream primer comprises SEQ ID NO: 12 or a variant thereof and the downstream primer comprises SEQ ID NO: 13 or a variant thereof and the strand invasion oligonucleotide comprises SEQ ID NO: 14 or a variant thereof;
(iv) the amplified target nucleic acid sequence comprises SEQ ID NO: 16, and the upstream primer comprises SEQ ID NO: 17 or a variant thereof and the downstream primer comprises SEQ ID NO: 18 or a variant thereof and the strand invasion oligonucleotide comprises SEQ ID NO: 19 or a variant thereof;
(v) the amplified target nucleic acid sequence comprises SEQ ID NO: 21, and the upstream primer comprises SEQ ID NO: 22 or a variant thereof and the downstream primer comprises SEQ ID NO: 23 or a variant thereof and the strand invasion oligonucleotide comprises SEQ ID NO: 24 or a variant thereof;
(vi) the amplified target nucleic acid sequence comprises SEQ ID NO: 26, and the upstream primer comprises SEQ ID NO: 27 or a variant thereof and the downstream primer comprises SEQ ID NO: 28 or a variant thereof and the strand invasion oligonucleotide comprises SEQ ID NO: 29 or a variant thereof;
(vii) the amplified target nucleic acid sequence comprises SEQ ID NO: 31, and the upstream primer comprises SEQ ID NO: 32 or a variant thereof and the downstream primer comprises SEQ ID NO: 33 or a variant thereof and the strand invasion oligonucleotide comprises SEQ ID NO: 34 or a variant thereof;
(viii) the amplified target nucleic acid sequence comprises SEQ ID NO: 36, and the upstream primer comprises SEQ ID NO: 37 or a variant thereof and the downstream primer comprises SEQ ID NO: 38 or a variant thereof and the strand invasion oligonucleotide comprises SEQ ID NO: 39 or a variant thereof;
(ix) the amplified target nucleic acid sequence comprises SEQ ID NO: 41, and the upstream primer comprises SEQ ID NO: 42 or a variant thereof and the downstream primer comprises SEQ ID NO: 43 or a variant thereof and the strand invasion oligonucleotide comprises SEQ ID NO: 44 or a variant thereof; or (x) the amplified target nucleic acid sequence comprises SEQ ID NO: 46, and the upstream primer comprises SEQ ID NO: 47 or a variant thereof and the downstream primer comprises SEQ ID NO: 48 or a variant thereof and the strand invasion oligonucleotide comprises SEQ ID NO: 49 or a variant thereof;
further wherein each said primer is an oligonucleotide of less than 30 nucleotides in length and wherein each strand invasion oligonucleotide is an oligonucleotide of more than 30 nucleotides in length, and wherein a said variant of a primer has at least 70% sequence identity to the sequence of the corresponding original sequence, and wherein a said variant of a strand invasion oligonucleotide comprises a region complementary to said target nucleic acid sequence having at least 70% sequence identity to the region complementary to said target nucleic acid sequence of the original strand invasion oligonucleotide, or comprises a complementary region to the region complementary to said target nucleic acid sequence of the original strand invasion oligonucleotide.

In a second aspect, the present invention provides a composition comprising (a) an upstream primer, (b) a downstream primer and (c) a strand invasion oligonucleotide, as defined in the method of the first aspect of the invention.

In a third aspect, the present invention provides a kit comprising (a) an upstream primer, (b) a downstream primer and (c) a strand invasion oligonucleotide, wherein each said oligonucleotide is as defined in the method of the first aspect of the invention.

In a fourth aspect, the present invention provides a method for diagnosis of an infection by a pathogen in a subject, comprising carrying out a method of the invention in a sample from said subject.

The present invention relates to detection of a target nucleic acid sequence of an RNA in a sample. The method of the invention for detecting a target nucleic acid sequence present in an RNA uses a reverse transcriptase and an upstream primer, a downstream primer, and a strand invasion oligonucleotide, each comprising a region complementary to said target nucleic acid sequence. The reverse transcriptase synthesises a complementary DNA from the RNA. In combination, the primers and strand invasion oligonucleotide provide for amplification of the target nucleic acid sequence from duplex nucleic acids formed in the method. The strand invasion oligonucleotide renders at least a portion of the target nucleic acid sequence single-stranded to allow the binding of the upstream primer and downstream primer, thereby permitting amplification. Typically, the amplification is performed under isothermal conditions, without a requirement for thermal denaturation of duplex nucleic acids. The reverse transcription of RNA to cDNA is typically carried out simultaneously with strand-invasion based amplification and detection of duplex nucleic acids comprising the target nucleic acid sequence derived from the starting RNA template.

The inventors have shown that the method of the invention allows for highly specific and sensitive detection of RNA targets, such as genomic RNA of influenza virus. Furthermore, the method of the invention is advantageous compared to the conventional RT-PCR method used for detection of RNA in various respects. For example, it can provide for improved sensitivity, reduced detection time and be less susceptible to sample-derived inhibition.

The inventors have further identified particularly effective target nucleic acid sequences for detection of influenza A and B employing the methods of the invention, and particularly effective primers and strand invasion oligonucleotides for use in amplification of these target sequences. The above target sequences, primers and oligonucleotides may also be used for detection of cDNA previously prepared from samples containing influenza A and B RNA.

The disclosure provides a method for detecting a ribonucleic acid (RNA) comprising a target nucleic acid sequence in a sample, said method comprising contacting said sample with a reverse transcriptase, at least one upstream primer, at least one downstream primer and at least one strand invasion oligonucleotide under conditions promoting amplification of said target nucleic acid sequence,
wherein each said primer and said strand invasion oligonucleotide comprises a region complementary to said target nucleic acid sequence;
wherein said strand invasion oligonucleotide renders at least a portion of a duplex nucleic acid comprising said target nucleic acid sequence single-stranded, to allow the binding of said upstream primer and a downstream primer.

The RNA comprising the target nucleic acid sequence is typically from an Influenza virus.

The disclosure further provides a method for detecting a target nucleic acid sequence from an influenza A or B gene in a sample, said method comprising contacting said sample with at least one upstream primer, at least one downstream primer and at least one strand invasion oligonucleotide under conditions promoting amplification of said target nucleic acid sequence,
wherein each said primer and said strand invasion oligonucleotide comprises a region complementary to said target nucleic acid sequence;
wherein said strand invasion oligonucleotide renders at least a portion of the target nucleic acid sequence single-stranded to allow the binding of said upstream primer and a downstream primer.

Typically said target sequence is from segment 1, 3, or 7 of influenza A or B gene said target sequence is from segment 1, 3, or 7 of Influenza A or segment 3 of Influenza B. Preferably the amplified target nucleic acid sequence is SEQ ID NO:1 or SEQ ID NO:6 or a variant of either thereof.

The disclosure additionally provides a composition or a kit, the composition or kit comprising at least two oligonucleotides selected from (a) an upstream primer, (b) a downstream primer and (c) a strand invasion oligonucleotide,
wherein said upstream primer is an oligonucleotide of less than 30 nucleotides in length comprising the sequence of SEQ ID NO: 2 or a variant thereof, said downstream primer is an oligonucleotide of less than 30 nucleotides in length comprising the sequence of SEQ ID NO: 3 or a variant thereof, and said strand invasion oligonucleotide is an oligonucleotide of greater than 30 nucleotides in length comprising the sequence of SEQ ID NO: 4 or a variant thereof, and further comprising one or more modified nucleotides in its 3'region; or
wherein said upstream primer is an oligonucleotide of less than 30 nucleotides in length comprising the sequence of SEQ ID NO: 7 or a variant thereof, and/or wherein said downstream primer is an oligonucleotide of less than 30 nucleotides in length comprising the sequence of SEQ ID NO: 8 or a variant thereof, and/or wherein said strand invasion oligonucleotide is an oligonucleotide of greater than 30 nucleotides in length comprising the sequence of SEQ ID NO: 9 or a variant thereof, and further comprising one or more modified nucleotides in its 3'region.

The disclosure also provides a method for diagnosis of an infection by a pathogen in a subject, comprising carrying out a method of the invention for detecting a ribonucleic acid (RNA) comprising a target nucleic acid sequence of a pathogen in a sample from said subject. Preferably, the pathogen is an Influenza virus. The disclosure also provides a method for diagnosis of an infection by Influenza A or B virus in a subject, comprising carrying out a method of the invention for detecting a target nucleic acid sequence from an influenza A or B gene in a sample from said subject.

### Brief Description of the Figures

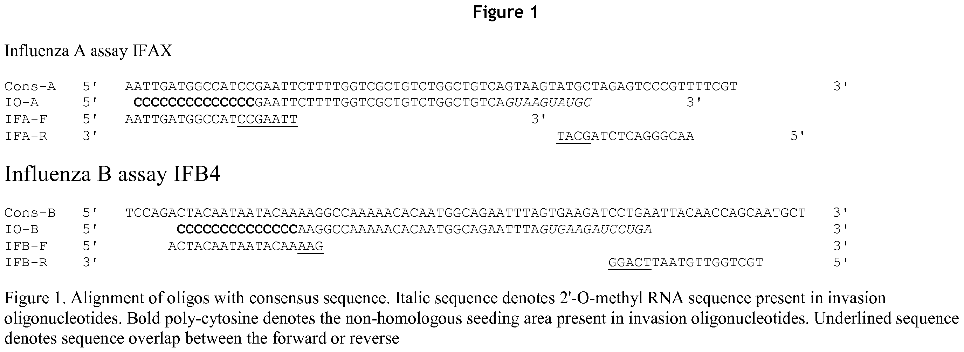

Figure 1 shows alignment of oligonucleotides used for SIBA with consensus sequences for Influenza A and B described in Example 1. Italic sequence denotes 2'-O-methyl RNA sequence present in invasion oligonucleotides. Bold poly-cytosine denotes the non-homologous seeding area present in invasion oligonucleotides. Underlined sequence denotes sequence overlap between the forward or reverse primers and the respective invasion oligonucleotide.

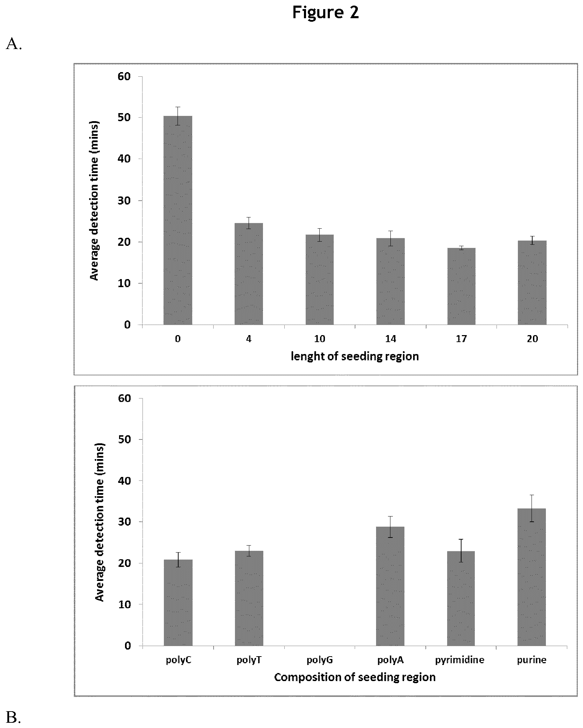

Figure 2 shows impact of the nucleotide composition of IO seeding region on fluA reaction speed. Panels A and B show average detection time (y-axis) for template RNA in an RT-SIBA assay using otherwise identical invasion oligonucleotides in which the length or nucleotide composition of the 5' seeding region is varied as shown (x-axis). A 14 nucleotide seeding region was used in the results shown in panel B.

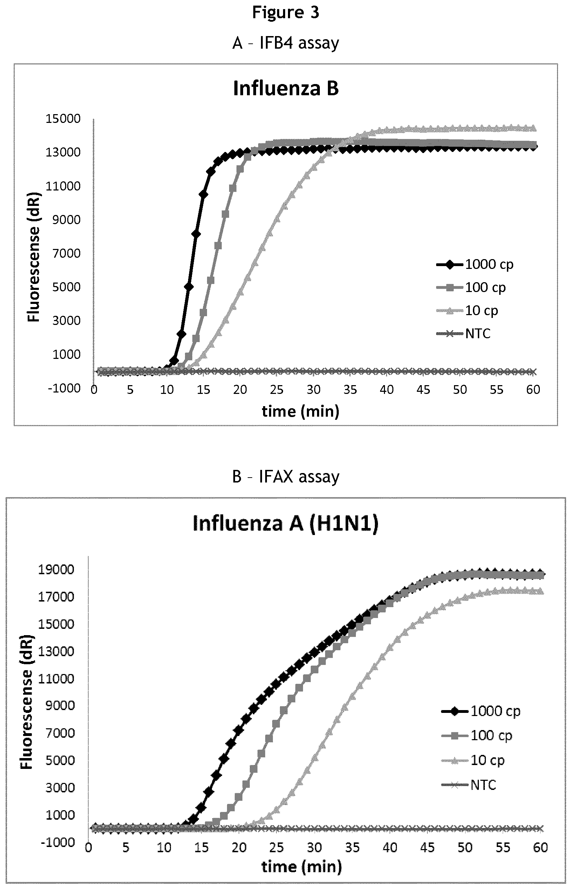

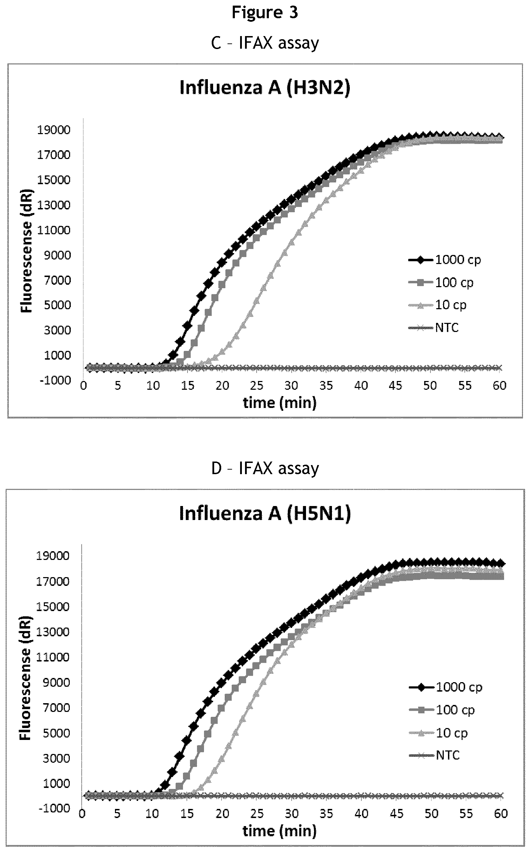

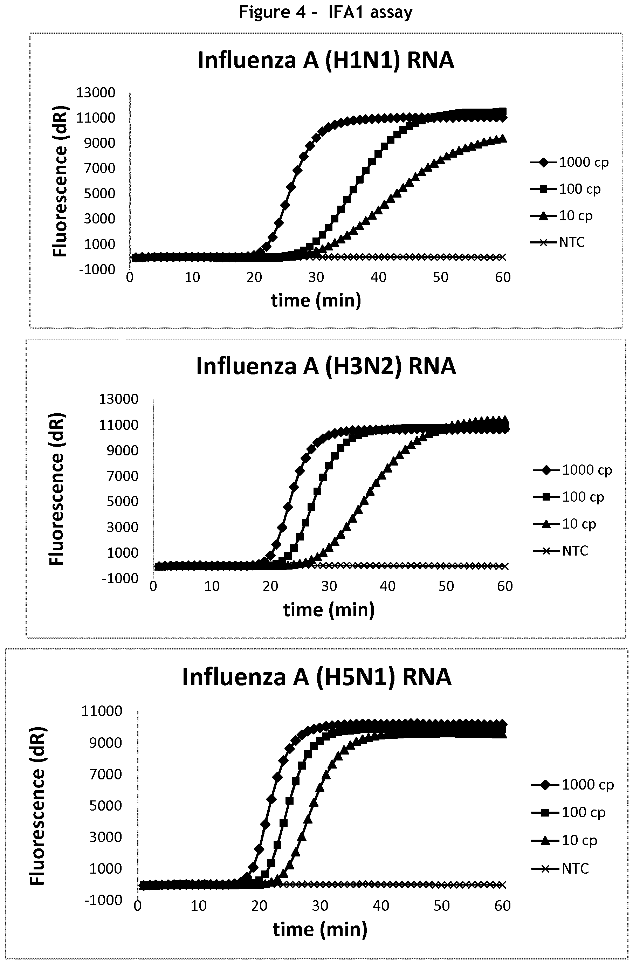

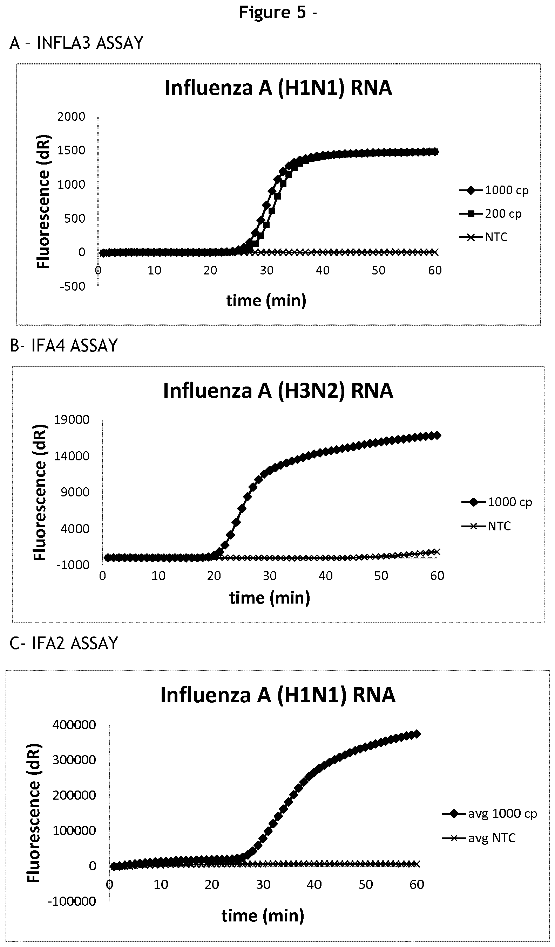

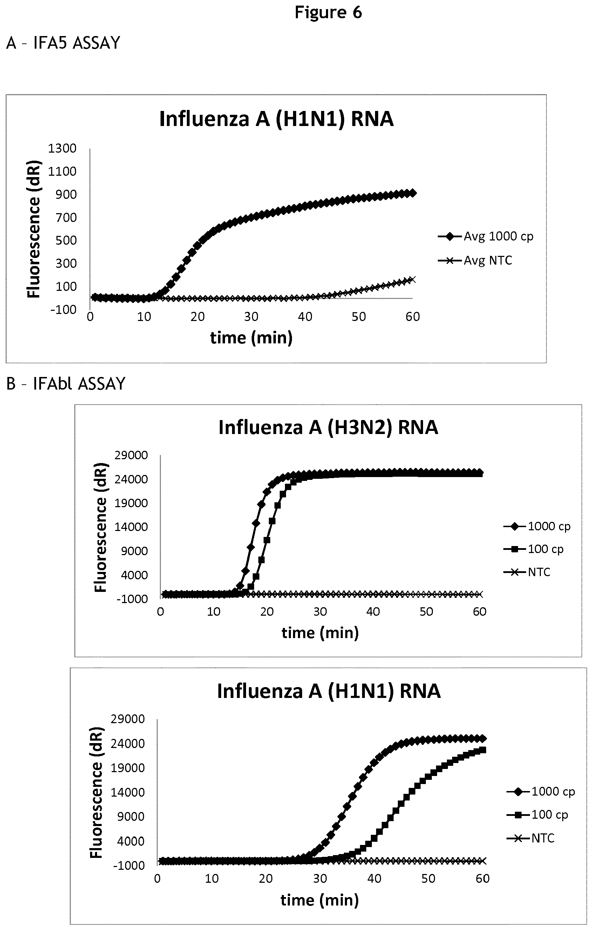

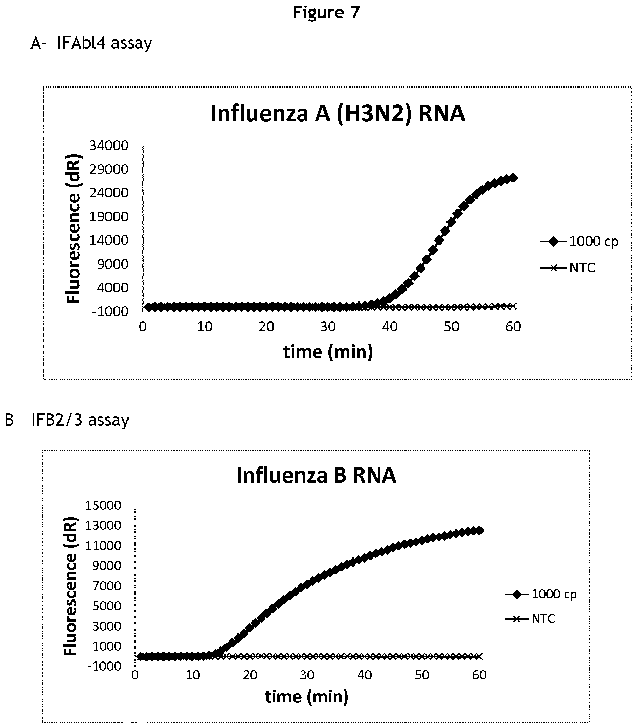

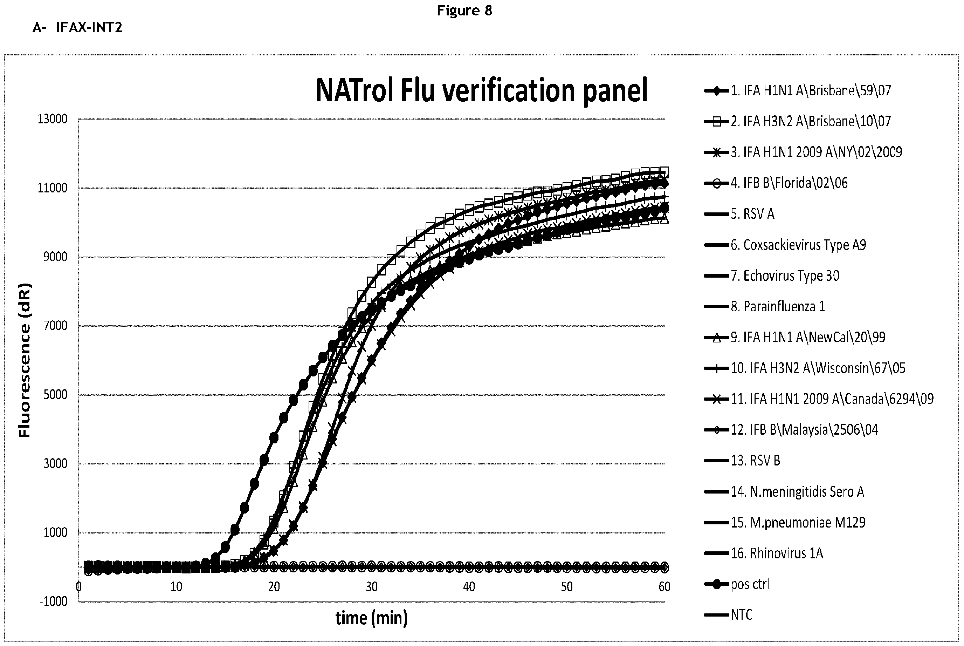

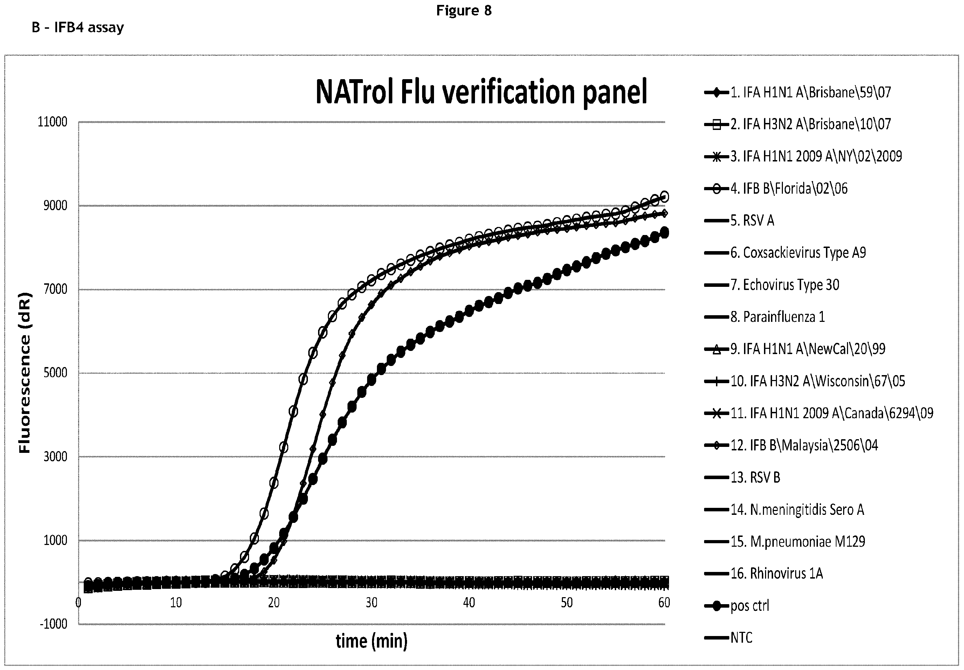

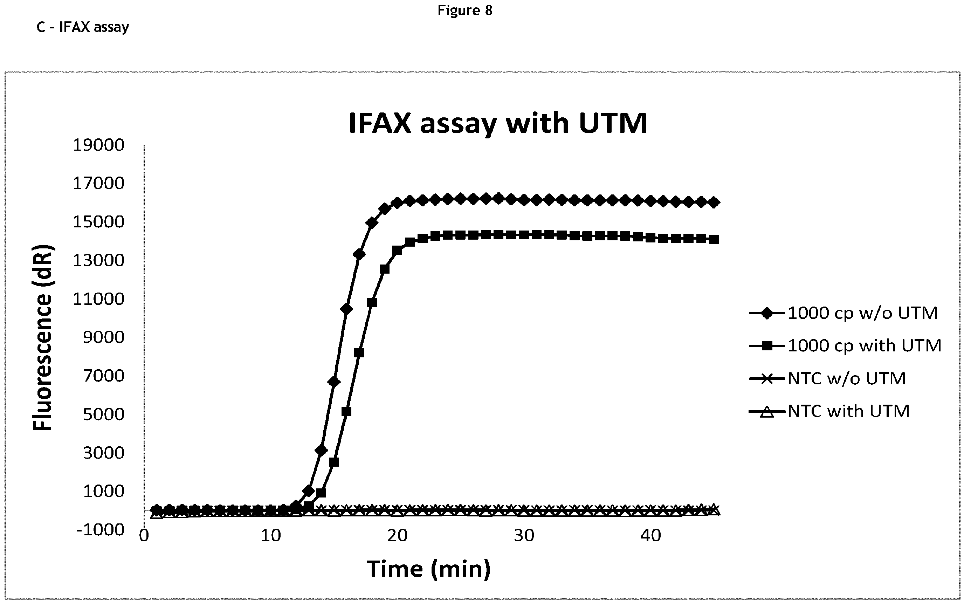

Figures 3-8 show sensitivity and inclusivity of influenza A and B RT-SIBA assays. Serial dilutions of RNA from different influenza serotypes were prepared and used as template in RT-SIBA assays. Assays based on amplification of various target regions were performed. Figures 3A and 7C show results for the IFB4 assay for Influenza B. Figures 3B-D and 8 show results for the IFAX assay for different subtypes of Influenza A. Figure 4 shows results for the IFA1 assay for different subtypes of Influenza A. Figure 5A shows results for the INFLA3 assay for Influenza A. Figure 5B shows results for the IFA4 assay for Influenza A. Figure 5C shows results for the IFA2 assay for Influenza A. Figure 6A shows results for the IFA5 assay for Influenza A. Figure 6B shows results for IFAbl assays for different subtypes of Influenza A. Figure 7A shows results for the IFAbl4 assay for Influenza A. Figure 7B shows results for the IFB2/3 assay for Influenza B.

The reactions were run in quadruplicate in three individual experiments. The figures show data from one experiments and the data is plotted as average from four replicate reactions. Total copy number of template RNA per reaction was 10 - 1000 cp/reaction. Y- axis: SybrGreen 1 fluoresence intensity (arbitrary units); X-axis: time (minutes). The results indicated that RT-SIBA could be used to reproducibly detect as low as 10 copies of influenza RNA per reaction. NTC = no template control.

Figures 8A and B show detection of various influenza A and B strains in a clinical sample panel. Figure 8A shows use of the IFAX assay, and Figure 8C shows the IFB4 5 assay. Y- axis: SybrGreen 1 fluorescence intensity (arbitrary units); X-axis: time (minutes).
NTC = no template control. Figure 8C shows detection of influenza A in the UTM clinical sample medium. Total copy number of template RNA per reaction was 1000 cp/reaction. Y- axis: SybrGreen 1 fluoresence intensity (arbitrary units); X-axis: time (minutes).

### Detailed Description of the Invention

It is to be understood that different applications of the disclosed methods may be tailored to the specific needs in the art. It is also to be understood that the terminology used herein is for the purpose of describing particular embodiments of the invention only, and is not intended to be limiting. In addition as used in this specification and the appended claims, the singular forms "a", "an", and "the" include plural referents unless the content clearly dictates otherwise. Thus, for example, reference to "a polypeptide" includes two or more such polypeptides, and the like.

### Methods of detection

### Sample

In embodiments where reverse transcription is carried out, the sample comprises RNA. If the RNA is present in the sample in a suitable form allowing for detection according to the invention, the sample may be used directly. Alternatively, the sample may be processed only by heating or treatment with detergents. However, the nucleic acid may also be derived, obtained or extracted from the sample. Methods for processing samples containing nucleic acids, extracting nucleic acids and/or purifying nucleic acids for use in detection methods are well-known in the art. Total nucleic acid may be isolated or RNA may be isolated separately.

Typically, a sample is processed in an appropriate manner such that nucleic acid is provided in a convenient form for contacting with the reverse transcriptase, primers and strand invasion oligonucleotide.

Commonly, the sample is a clinical sample, for example a sample obtained from a patient suspected of having, or having an infection by a pathogen, in particular a viral pathogen. The viral pathogen is preferably an influenza virus. The RNA may be genomic RNA of a virus. The RNA may also be bacterial mRNA. RNA may also be useful for bacterial detection, owing to the very large number of ribosomes present in bacterial cells which effectively amplify the concentration of target sequences. The RNA is typically single-stranded RNA but double-stranded RNA may also be used.

However, any sample can be used, provided that RNA can be obtained or derived from the sample. Thus, reference samples (for example for particular pathogens/particular viral strains), or environmental samples may be used in the present invention. Suitable types of clinical sample vary according to the particular type of infection that is present, or suspected of being present in a subject. The sample may be blood, plasma, saliva, serum, sputum, urine or a stool sample. Influenza samples may be collected by nose-throat swab from nasal and pharyngeal cavity. The sample may be stored in universal transport media (UTM).

In preferred embodiments, the samples are taken from animal subjects, such as mammalian subjects. The samples will commonly be taken from human subjects, but the present invention is also applicable in general to domestic animals, livestock, birds and fish. For example, the invention may be applied in a veterinary or agricultural setting.

In embodiments where reverse transcription of RNA has previously been carried out, a suitable sample of cDNA or dsDNA amplified from the starting RNA is provided as a template.

### Reverse transcription

The RNA from the sample is contacted with a reverse transcriptase or any other enzyme (such as a polymerase) having reverse transcriptase activity under conditions promoting reverse transcription of the RNA into cDNA. Suitable conditions include any conditions used to provide for activity of reverse transcriptase enzymes known in the art. The conditions typically include the presence of one or more species of primer, all four dNTPs, dATP, dTTP, dCTP and dGTP or analogues thereof, suitable buffering agents/pH and other factors which are required for enzyme performance or stability. The primer may be oligo d(T). A mixture of random primers (such as random hexamers) may be used. Alternatively, the same primers to be used for amplification of the target nucleic acid sequence may be used also for priming reverse transcription of the RNA.

The conditions may also include the presence of detergents and stabilising agents. The conditions may also include additional proteins. Preferably the additional proteins may be single-strand DNA binding proteins. The temperature used is typically isothermal, i.e constant throughout the reverse transcription process. Preferably, the temperature is identical to use for amplification of the target nucleic acid sequence, such that reverse transcription and amplification occur simultaneously in the same reaction temperature, without need for temperature cycling (as in RT-PCR), This provides significant benefits for speed of detection. The temperature used typically depends on the nature of the reverse transcriptase enzyme and other enzyme components, and also reflects the hybridisation temperature required for the primer(s).

Suitable reverse transcriptases include Moloney Murine Leukemia Virus Reverse Transcriptase (M-MLV RT) and functional fragments or variants thereof. Particular suitable reverse transcriptases include Improm, GoScript, MMuLV, AMV, RevertAid transcriptases.

### Target nucleic acid sequence

The target nucleic acid sequence is a region (or amplicon) suitable for detection by strand-invasion based amplification. The selection of suitable target nucleic acid sequences, and the consequent design of primer and strand invasion oligonucleotides for detection of those sequences is an important consideration. Examples of appropriate sequences are provided herein. The target nucleic acid sequences exemplified herein are presented as DNA sequences. However, it should be understood that any DNA sequence described herein also encompasses its RNA equivalent. Thus for example the target sequence of SEQ ID NO: 1 has as its RNA equivalent SEQ ID NO: 61.. Accordingly, where an RNA is detected in accordance with the invention, this RNA will include the target nucleic acid sequence in RNA form. This RNA sequence will be reverse transcribed to cDNA and then subject to amplification, such that the target nucleic acid sequence which acts as the template for the primers and strand invasion oligonucleotide will typically be in DNA form.

Typically, where a pathogen is to be detected, the target nucleic acid sequence will be unique to its genome. This allows for a highly qualitative, unambiguous determination of the presence of the pathogen in the sample, even if closely related organisms exist. The target nucleic acid sequence will thus typically differ from any homologous nucleic acid sequence in a related species. Typically, the target nucleic acid sequence will comprise several mismatches with a homologous nucleic acid sequence in a related species.

Specific aspects of the invention relate to detection of influenza viruses. Preferably, the target nucleic acid sequence is not present in any other type of virus, or not present in any other respiratory virus. More preferably, the target nucleic acid sequence is specific to a particular serotype of influenza. The known influenza serotypes include Influenza A, B and C. Preferably, the target nucleic acid sequence is specific for Influenza A or Influenza B. The target nucleic acid sequence may be specific for a particular strain or subtype of Influenza A or B. Preferably, the target nucleic acid sequence is specific for a pathogenic subtype of influenza A or B. For example, the target nucleic acid sequence may be specific for Influenza A subtype H1N1, H3N2 or H5N1. Influenza subtypes are named according to hemagglutinin type (H) and neuraminidase type (N). Alternatively, the target nucleic acid sequence may be inclusive for multiple different subtypes or strains, and thus is typically present and can be detected in more than one subtype or strain. Preferably, the target nucleic acid sequence is inclusive for at least two or three, more preferably at least five, at least seven, at least ten different strains or subtypes, typically pathogenic strains or subtypes. Typically, the target nucleic acid sequence is inclusive for the most clinically relevant subtypes or strains , in respiratory illnesses caused by Influenza A or Influenza B subtypes. The target nucleic acid sequence may be inclusive preferably for Influenza A subtypes H1N1, H3N2 and H5N1. The target nucleic acid sequence may also be inclusive for Influenza B.

The target nucleic acid sequence or amplicon is of a sufficient length to provide for hybridisation of the upstream and downstream primers and strand invasion oligonucleotide in a suitable manner to different portions of the target sequence. Preferably, the amplicon is at least 45 nucleotides in length, more preferably at least 50, at least 55, at least 60, or at least 65 nucleotides in length, as measured from the 5' site of binding of the upstream primer to the 5' site of binding of the downstream primer. The amplicon may be about 45 to about 70, about 50 to about 70, about 55 to about 70 or about 60 to about 70 nucleotides in length. In some instances, an amplicon may be selected which is to be invaded at two separate upstream and downstream locations by one or more strand invasion oligonucleotides, as described in WO/2015/185655. This permits use of longer amplicons, such as those of about 70 to about 500 nucleotides in length, about 70 to about 400, about 100 to about 400, or about 100 to about 200 nucleotides in length.

In the preferred aspects related to detection of Influenza virus, the target nucleic acid sequence may be present in any region of the viral genome, provided it has the necessary characteristics for specific detection of the virus as discussed above. Preferably, the target nucleic acid sequence is present in segment 1, 3, or 7.

The target nucleic acid sequence preferably comprises SEQ ID NO: 1 or a variant thereof (for detection of Influenza A) or SEQ ID NO: 6 or a variant thereof (for detection of Influenza B). It should be understood that the target nucleic acid sequence which provides the template for amplification will be present in a duplex which comprises a sense strand representing SEQ ID NO:1 or a variant thereof, or SEQ ID NO: 6 or a variant thereof, and a complementary anti-sense strand. The upstream and downstream primers used to amplify the target nucleic acid sequence bind to opposing strands of this duplex.

The target nucleic acid sequence may comprise a naturally occurring variant sequence of SEQ ID NO:1 or SEQ ID NO:6 which is present in a different subtype or strain.

Variants of SEQ ID NO: 1 or SEQ ID NO: 6 may comprise a region which is partly or fully complementary or identical to at least 35 contiguous nucleotides, more typically at least 40 or at least 45 contiguous nucleotides, preferably at least 50 or at least 55 contiguous nucleotides of SEQ ID NO: 1 or SEQ ID NO: 6. The variants may comprise a region which has 1, 2, 3, 4, 5, 6, 7, 8, 9, 10, 11, 12 or more mismatches (substitutions) with respect to a region of the corresponding original target sequence of SEQ ID NO: 1 or SEQ ID NO: 6.

Thus, for instance the variants may comprise a region of at least 35 nucleotides in length which has 1, 2, 3, 4, 5, or 6 mismatches, such as 1-3 or 1-5 mismatches, to a corresponding region of at least 35 contiguous nucleotides of the corresponding original target sequence. The variants may comprise a region of at least 40, 45, or 50 nucleotides in length which has 1, 2, 3, 4, 5, 6, 7, 8, 9 or 10, such as 1-5 or 1-8 mismatches to a corresponding region of an equivalent length in the corresponding original target sequence. Any mismatches in the variant sequence may be at least 2, at least 4, at least 5, or at least 10 nucleotides apart.

Alternatively, the variants may comprise a region of at least 35, 40, or 45 nucleotides in length which is in full complementarity or identity with the original target sequence.

Preferably, the variants comprise only one or two mismatches to SEQ ID NO: 1 or 6 in a region corresponding to the strand-invasion oligonucleotide-complementary region of SEQ ID NO: 1 or SEQ ID NO:6. More preferably, the variants comprise a region which is in full complementarity or identity with the strand-invasion oligonucleotide complementary region of SEQ ID NO:1 or SEQ ID NO:6.

Most preferably, the target nucleic acid sequence comprises SEQ ID NO: 1 or SEQ ID NO: 6 or consists of SEQ ID NO: 1 or SEQ ID NO: 6 and a complementary antisense strand.

Other target nucleic acid sequences that may be detected in accordance with the invention include SEQ ID NOs 11, 16, 21, 26, 31, 36 and 41 (from Influenza A) and SEQ ID NO: 46 (from Influenza B). Thus, the target nucleic acid sequence to be detected may comprise any of SEQ ID NOs 11, 16, 21, 26, 31, 36 and 41 or comprise a variant of any thereof. Variants of SEQ ID NOs 11, 16, 21, 26, 31, 36 and 41 may be selected according to the same criteria set out above in relation to SEQ ID NOs 1 and 6.

Additional variant target nucleic acid sequences provided herein are the longer consensus sequences from which the above amplicons are derived, as described below. Thus, the consensus sequences of any of SEQ ID NOs 51 to 60 or fragments thereof may be amplified in accordance with the invention.

More than one target nucleic acid sequence may be detected in a method of the invention, by providing two or more sets of upstream primer, downstream primer and strand invasion oligonucleotide, each set adapted for detection of a different target nucleic acid sequence. For example, a method of the invention may detect both influenza A and influenza B.

### Upstream and downstream primers

Suitable upstream and downstream primers are selected based on the target nucleic acid sequence of interest, and having regard to the site of binding of the strand invasion oligonucleotide that renders at least a portion of the target nucleic acid sequence single-stranded to allow the binding of the upstream primer and downstream primer.

The upstream and downstream primers comprise a sequence that is partly or fully complementary or identical to the target and optionally a 5' and/or 3' flanking non-complementary sequence. Alternatively, the upstream and downstream primers may consist entirely of partly or fully complementary or identical sequence to the target. The length of the primer sequence that is complementary or identical to the target is sufficient to provide specific hybridisation to the target nucleic acid sequence. The length of complementary or identical sequence is typically at least 10 nucleotides, more preferably at least 15, at least 16, or at least 17 nucleotides. The length of complementary or identical sequence maybe 10-25, 15-25, 10-30 or 15-30 nucleotides.

It should be understood that the above sequence lengths refer to portions of the primers which may be partly or fully complementary or identical to the target nucleic acid sequence. Mismatches may be present between the primers and the target sequence at particular positions while still allowing for specific amplification and detection of the target sequence, in particular having regard to the combined use of upstream and downstream primers and a strand invasion oligonucleotide to achieve amplification. There may be 1, 2, 3, 4 or 5 mismatches between the complementary or identical region of the primer and the corresponding region of the target sequence.

Preferably, the primer is designed to allow for specific detection of a pathogen of interest (typically an Influenza virus). Thus, the primer typically specifically or selectively hybridises to a complementary sequence found only in the pathogen of interest. However, the primer may also hybridise to other sequences, such as sequences found in other pathogens, provided that when used in combination with the second primer and strand invasion oligonucleotide, specific amplification of a sequence found only in the pathogen of interest is obtained.

Specific or selective hybridisation refers to the binding of a primer only to a particular nucleotide sequence under given conditions, when that sequence is present in a nucleic acid in a sample, such as a complex biological mixture including total cellular and foreign DNA or RNA. Appropriate hybridisation conditions are known in the art. See for example, Sambrook, Fritsche and Maniatis "Molecular Cloning: A Laboratory Manual", 2nd Ed. Cold Spring Harbor Press (1989). Appropriate hybridisation conditions are also provided in the Examples below. As is known to the skilled person, appropriate hybridisation conditions may vary depending on the length of a primer and its base composition. Hybridisation is typically performed at the same temperature as amplification, and thus also depends on the activity profile of the polymerase and recombinase enzymes employed.

Typically the upstream and downstream primer will be less than 30 nucleotides in total in length, more preferably less than 25 nucleotides in length, such as 15 to 25, or 15 to 23 nucleotides in length. It is particularly preferred that primers of less than 30 nucleotides in length are used where a recombinase is used for strand invasion. The primers are not capable of acting as substrates for recombinases.

The upstream (or forward) primer binds to the 5' region of one strand of the duplex target nucleic acid sequence, at a position proximal or overlapping with the 5' binding site of the strand invasion oligonucleotide. The downstream (or reverse) primer binds to the 5' region of the opposing strand of the duplex target nucleic acid sequence to the upstream primer, at a position proximal or overlapping with the 3' binding site of the strand invasion oligonucleotide. The 5' binding sites of the upstream and downstream primers are typically at least 45 nucleotides, more preferably at least 50, at least 55, at least 60, or at least 65 nucleotides apart on the duplex target sequence.

The upstream and/or downstream primer may have a region of sequence overlap with the sequence of the strand invasion oligonucleotide. The region of sequence overlap is typically 1-8 nucleotides in length, preferably 4-6 nucleotides in length, and may be at least 3, at least 4, at least 5 or at least 6 nucleotides in length. The downstream primer may also have correspondingly defined regions of sequence overlap with the sequence of the strand invasion oligonucleotide.

Alternatively, there may be no sequence overlap between the upstream and/or downstream primer and the strand invasion oligonucleotide, with the primer binding instead at a position that is proximal in the target sequence to the binding site of the strand invasion oligonucleotide.

Where a primer binds proximal to the strand invasion oligonucleotide, typically there is 25 nucleotides or less, more preferably 20 nucleotides or less, 15 nucleotides or less, or 10 nucleotides or less between the relevant binding site of the strand invasion oligonucleotide and the 5' end of the primer. This ensures that the primer is able to hybridise to the single-stranded region created by binding of the strand invasion oligonucleotide.

Specific examples of suitable upstream and downstream primers for binding of target nucleotide sequences in Influenza genes are provided herein. Preferred upstream and downstream primers for detection of the target sequence of SEQ ID NO:1 are the primers of SEQ ID NOs 2 and 3, or variants thereof. Preferred upstream and downstream primers for detection of the target sequence of SEQ ID NO: 6 are the primers of SEQ ID NOs 7 and 8, or variants thereof.

Variants of SEQ ID NOs 2, 3, 7 and 8 may be oligonucleotides of up to 30 nucleotides in length comprising a region which is partly or fully complementary or identical to at least 10 contiguous nucleotides of the corresponding original primer sequence of SEQ ID NO: 2, 3, 7 or 8. Preferably, said variants will comprise a region which is partly or fully complementary or identical to at least 11, 12, 13, 14 or 15 contiguous nucleotides of the corresponding original primer sequence of SEQ ID NO: 2, 3, 7 or 8. Where the original primer sequence is longer than 16 nucleotides in length, such as up to 21 nucleotides in length (SEQ ID NO:2) the variants may correspondingly comprise a region which is partly or fully complementary or identical to 16, 17, 18, 19 or 20 contiguous nucleotides thereof.

The above variants may comprise a region which has 1, 2, 3, 4, or 5 mismatches (substitutions) with respect to the corresponding region of the original primer sequence (and thus the target sequence) and thus is partly complementary or identical thereto. Thus, for instance, the variants may comprise a region of at least 10 nucleotides in length which has 1, 2, or 3 mismatches, such as 1 or 2 mismatches to a corresponding region of at least ten contiguous nucleotides of the corresponding original primer sequence. The variants may comprise a region of at least 13, 14 or 15 nucleotides in length which has 1, 2, 3, 4 or 5 mismatches, such as 1-3 mismatches to a corresponding region of an equivalent length in the corresponding original primer sequence. Any mismatches in the variant primer sequence may be at least 2, at least 4, at least 5, or at least 10 nucleotides apart.

Alternatively, the variants may comprise a region of at least 10, 11, 12, 13, 14 or 15 nucleotides in length which is in full complementarity or identity with the original primer sequence.

Variants of SEQ ID NOs 2, 3, 7 and 8 may also be oligonucleotides of up to 30 nucleotides in length which have at least 70% sequence identity to the sequence of the corresponding original primer sequence, preferably at least 75%, at least 80%, more preferably at least 85%, at least 90%, at least 95% sequence identity.

Additionally, the variant primers may comprise region(s) complementary or identical to the 5' or 3' flanking nucleotide sequence(s) to the binding region of the original primers in the gene comprising the target nucleic acid sequence, such as 5-10 nucleotides from the 5' flanking region and/or 3-region. The variant primers may additionally comprise sequence unrelated to the target sequence.

For detection of the target nucleic acid sequence of SEQ ID NO:11, primers comprising the sequences of SEQ ID NOs 12 and 13 or variants thereof are additionally provided herein. Similarly, for the target nucleic acid sequence of SEQ ID NO: 16, primers comprising the sequences of SEQ ID NOs 17 and 18 or variants thereof are provided. Additionally, for the target nucleic acid sequence of SEQ ID NO: 21, primers comprising the sequences of SEQ ID NOs 22 and 23 or variants thereof are provided. Also, for the target nucleic acid sequence of SEQ ID NO: 26, primers comprising the sequences of SEQ ID NOs 27 and 28 or variants thereof are provided. For detection of the target nucleic acid sequence of SEQ ID NO: 31, primers comprising the sequences of SEQ ID NOs 32 and 33 or variants thereof are provided. Furthermore, for detection of the target nucleic acid sequence of SEQ ID NO: 36, primers comprising the sequences of SEQ ID NOs 37 and 38 or variants thereof are provided. Additionally, for detection of the target nucleic acid sequence of SEQ ID NO: 41 primers comprising the sequences of SEQ ID NOs 42 and 43 or variants thereof are provided. Finally, for detection of the target nucleic acid sequence of SEQ ID NO: 46 primers comprising the sequences of SEQ ID NOs 47 and 48 or variants thereof are provided.

All of the above primers are typically oligonucleotides of less than 30 nucleotides in length. Suitable variant sequences for the primers for the additional target sequences described above may be selected in accordance with the same detailed criteria described in relation to variants of the primers for SEQ ID NOs 1 and 6.

Any upstream or downstream primer used in the invention may comprise one or more modified nucleotides and/or a detectable label, for example a fluorescent dye.

### Strand invasion oligonucleotide

A suitable strand invasion oligonucleotide is selected based on the target nucleic acid sequence of interest, and having regard to the site of binding of the upstream and downstream primers and the requirement for the strand invasion oligonucleotide to render the target nucleic acid sequence single-stranded in the relevant regions to allow for the binding of the upstream primer and downstream primer.

The strand invasion oligonucleotide comprises a sequence that is complementary or identical to the target and optionally additional flanking non-complementary or non-identical sequence(s). The length of the sequence that is complementary or identical to the target may be determined by the skilled person empirically and is sufficient to provide for efficient strand invasion of the target nucleic acid sequence, optionally under isothermal conditions. The complementary sequence may comprise RNA-DNA complementary base pairing and modified nucleotides. Typically, the length of complementary or identical sequence is at least 25 or at least 27 nucleotides, typically at least 30 nucleotides, such as least 32, at least 33 or at least 35 nucleotides, more preferably at least 36, 37, 38, 39 or 40 nucleotides in length or greater. The length of complementary or identical sequence may be 30-50, 32-50, 35-50, 40-50, 35 to 48, 35 to 46, 38 to 45 or 40 to 45 nucleotides in length.

It should be understood that the above sequence lengths refer to a portion of the strand invasion oligonucleotide which may be partly or fully complementary or identical to the target nucleic acid sequence. Mismatches may be present between the strand invasion oligonucleotide and the target sequence at particular positions while still allowing for specific amplification and detection of the target sequence, in particular having regard to the combined use of upstream and downstream primers and a strand invasion oligonucleotide to achieve amplification. There maybe 1, 2, 3, 4, 5, 6, 7, or 8 mismatches between the complementary or identical region of the strand invasion oligonucleotide and the corresponding region of the target sequence, depending on the total length of complementary or identical sequence. Preferably though there is only 1 or 2 mismatches between the strand invasion oligonucleotide sequence and the corresponding region of the target sequence.

Preferably, the complementary or identical sequence of the strand invasion oligonucleotide is designed to allow for specific detection of a pathogen of interest (typically an Influenza virus). Thus, the strand invasion oligonucleotide preferably specifically or selectively hybridises to a complementary sequence found only in the pathogen of interest. However, the strand invasion oligonucleotide may also hybridise to other sequences, such as sequences found in other pathogens, provided that when used in combination with the primers, specific amplification of a sequence found only in the pathogen of interest is obtained.

The complementary or identical sequence of the strand invasion oligonucleotide hybridises to a portion of the target sequence intervening the binding regions for the upstream and downstream primers (and typically overlapping with one or more thereof). The strand invasion oligonucleotide may have a region of overlap of 1-8 nucleotides, preferably 4-6 nucleotides in length, such as a region of at least 3, at least 4, at least 5 or at least 6 nucleotides in length, with the upstream and/or downstream primers.

The 5' portion of the complementary or identical sequence of the strand invasion oligonucleotide typically binds within 25 nucleotides or less, more preferably 20 nucleotides or less from the 5' boundary of the duplex target nucleotide sequence to be melted (the amplicon).

The strand invasion oligonucleotide optionally further comprises non-complementary or non-identical sequence region(s) to the target that flank the complementary or identical sequence region. The strand invasion oligonucleotide may comprise a non-complementary or non-identical 5' region which may be of any nucleotide sequence. The 5' non-complementary region may assist binding of recombinase and is also referred to herein as the "seeding" region.

The 5' non-complementary region is typically at least 3 nucleotides in length, more typically at least 6, at least 8, at least 10 nucleotides in length. More preferably, the 5' non-complementary region is of at least 12, most preferably at least 14 nucleotides in length. The 5'non-complementary region may be greater than 14 nucleotides in length and may be up to 20 nucleotides in length. The 5' non-complementary region may be 10-20, 10-17, 10-14, 12-20, or 12-17 nucleotides in length, more preferably about 14 to about 20 or about 14 to about 17 nucleotides in length. The 5' non-complementary region may be of any nucleotide composition but preferably is selected to have particular nucleotide compositions described herein as being advantageous in the invention. Preferably, the 5' non-complementary region comprises or consists essentially of pyrimidine (cytosine or guanine) nucleotides. The non-complementary region may comprise, consist or consist essentially of a mixture of cytosine and guanine nucleotides. Preferably, where a mixture of pyrimidine nucleotides is present, a greater number of cytosine residues than guanine residues is present. Most preferably, the non-complementary region consists essentially of, or consists of, cytosine nucleotides. In preferred embodiments, the 5'non-complementary region is of about 14 to about 20 nucleotides in length and comprises, consists or consists essentially of a mixture of cytosine and guanine nucleotides. In particularly preferred embodiments, the 5'non-complementary region is of about 14 to about 20 nucleotides in length and comprises, consists or consists essentially of cytosine nucleotides. In especially preferred embodiments, the 5'non-complementary region is about 14 nucleotides in length and consists or consists essentially of cytosine residues.

The strand invasion oligonucleotide may comprise a 3' non-complementary region typically of 1-3 nucleotides in length which comprises nucleotides which block polymerase extension such as invdT.

The strand invasion oligonucleotide is typically at least 30 nucleotides in length where a recombinase is used in conjunction with the oligonucleotide. The strand invasion oligonucleotide is preferably at least 35, at least 40 or at least 45 nucleotides in length, more preferably at least 50, and may be at least 55 nucleotides in length or greater. The strand invasion oligonucleotide may be 40-70, 45-70, 45-70, 50-70, 55-70, 45-65, 50-65, 50-60 or 55-65 nucleotides in length.

Typically the strand invasion oligonucleotide has a non-extendible 3'terminus, such that it cannot serve as a substrate for DNA amplification, and the target sequence is then only amplified on the further binding of the specific upstream and downstream primers. This avoids formation of non-specific amplification products. The strand invasion oligonucleotide may comprise one, two, three, four, five, six, seven, eight or more modified nucleotides in its 3'region, such as in the 10-15 or 10-20 nucleotides from the 3'terminus. The strand- invasion oligonucleotide may comprise a 3' modification of the 3'terminal nucleotide, and may be a dideoxynucleotide, or comprise a 3'amino-allyl group, a 3'carbon spacer, 3'phosphate, 3'biotin, 3'sialyl, or 3'thiol. The 3' nucleotide may be a nucleotide incorporated in a reversed orientation by a 3'-3' linkage. Alternatively or additionally, the 3' region of the strand-invasion oligonucleotide may comprise nucleotides with poor substrate capability for DNA polymerases, such as PNA (peptide nucleic acid) nucleotides, LNA (locked nucleic acid), 2'-5' linked DNA or 2'-O-methyl RNA, or combinations thereof.

Where the strand-invasion oligonucleotide is a PNA oligomer comprised wholly of PNA, such an oligonucleotide can destabilise and invade duplex DNA in the absence of a recombinase enzyme. Thus, where a PNA oligonucleotide is used, the methods of the invention may be performed without presence of a recombinase enzyme.

Specific examples of suitable strand invasion oligonucleotides for target nucleotide sequences in Influenza genes are provided herein. A preferred strand invasion oligonucleotide for detection of the target sequence of SEQ ID NO: 1 is SEQ ID NO: 4. A particularly preferred strand invasion oligonucleotide is a modified derivative of SEQ ID NO: 4, most preferably SEQ ID NO: 5. A preferred strand invasion oligonucleotide for detection of the target sequence of SEQ ID NO: 6 is SEQ ID NO: 9. A particularly preferred strand invasion oligonucleotide is a modified derivative of SEQ ID NO: 9, most preferably SEQ ID NO: 10.

As discussed above, it is preferred that a strand invasion oligonucleotide used in the invention comprises one or more modified oligonucleotides in its 3'region to block its use as a polymerase substrate. Thus, a modified derivative of SEQ ID NO: 4 or 9 may comprise one, two, three, four, five, six, seven, eight or more modified nucleotides in its 3'region, typically in the 10-15 or 10-20 nucleotides from the 3'terminus. The modifications may be selected from any of those discussed above. The modified derivative may be a PNA oligomer of corresponding sequence to SEQ ID NO: 4 or 9.

In addition to modified derivatives of SEQ ID NOs 4 and 9, variant strand invasion oligonucleotides may be used.

Variants of SEQ ID NOs 4 and 9 are typically oligonucleotides of greater than 30 nucleotides, more preferably at least 35, at least 40, or at least 45 nucleotides in length, comprising a region which is partly or fully complementary or identical to at least 30 contiguous nucleotides of the corresponding original target-binding sequence present in SEQ ID NO: 4 or 9. The target-binding sequence of SEQ ID NOs: 4 and 9 is the region which has complementarity or identity to the target.

Preferably, said variants will comprise a region which is partly or fully complementary or identical to at least 32, 35, 37, 40, 42 or 45 contiguous nucleotides of the target-binding sequence present in SEQ ID NO: 4 or 9.

The above variants may comprise a region which has 1, 2, 3, 4, 5, 6, 7 or 8 mismatches (substitutions) with respect to the corresponding target-binding region of the original strand invasion oligonucleotide of SEQ ID NO: 4 or 9 (and thus the target sequence) and thus is partly complementary or identical thereto. Thus, for instance, the variants may comprise a region of at least 30 nucleotides in length which has 1, 2, 3, or 4, such as 1-4 or 1-3 mismatches to a corresponding region of at least 40 contiguous nucleotides of the corresponding original strand invasion oligonucleotide. The variants may comprise a region of at least 35, 40, 42, or 45 nucleotides in length which has 1, 2, 3, 4, 5 or 6, such as 1-5, or 1-3 mismatches to a corresponding region of an equivalent length in the corresponding original strand invasion oligonucleotide. Any mismatches in the variant strand invasion oligonucleotide sequence may be at least 2, at least 4, at least 5, or at least 10 nucleotides apart.

Alternatively, the variants may comprise a region of at least 32, 35, 37, 40, 42 or 45 nucleotides in length which is in full complementarity or identity with the target-binding region of the original strand invasion oligonucleotide.

Variants of SEQ ID NOs 4 and 9 may also be oligonucleotides of greater than 30 nucleotides in length comprising a target-binding region which has at least 70% sequence identity to the target-binding sequence of the corresponding original strand invasion oligonucleotide, preferably at least 75%, at least 80%, more preferably at least 85%, at least 90%, at least 95% sequence identity.

Additionally, the variant strand invasion oligonucleotides may comprise additional sequence(s) complementary or identical to the 5' and/or 3' flanking nucleotide sequence(s) to the binding region for the original strand invasion oligonucleotide in the gene from which the target nucleic acid sequence is derived, such as 5-10 or 5-15 nucleotides from the 5' flanking region and/or 3-region.

The remaining sequence of the variant strand invasion oligonucleotides is typically unrelated to the target sequence, and also typically unrelated to the original strand invasion oligonucleotide.

The variant strand invasion oligonucleotides further comprise one or more modified oligonucleotides in their 3'region such as, two, three, four, five, six, seven, eight or more modified nucleotides, which may be in the 10-15 or 10-20 nucleotides from the 3'terminus The modifications may be selected from any of those discussed above.

For detection of the target nucleic acid sequence of SEQ ID NO:11, a strand invasion oligonucleotide comprising SEQ ID NOs 14 or a variant or modified derivative thereof is provided herein. The modified derivative is preferably SEQ ID NO:15. Similarly, for the target nucleic acid sequence of SEQ ID NO: 16, a strand invasion oligonucleotide comprising SEQ ID NOs 19 or a variant or modified derivative thereof is provided. The modified derivative is preferably SEQ ID NO:20. Additionally, for the target nucleic acid sequence of SEQ ID NO: 21, a strand invasion oligonucleotide comprising SEQ ID NO 24 or a variant or modified derivative thereof is provided. The modified derivative is preferably SEQ ID NO:25. Also, for the target nucleic acid sequence of SEQ ID NO: 26, a strand invasion oligonucleotide comprising SEQ ID NO 29 or a variant or modified derivative thereof is provided. The modified derivative is preferably SEQ ID NO: 30. For detection of the target nucleic acid sequence of SEQ ID NO: 31, a strand invasion oligonucleotide comprising SEQ ID NO 34 or a variant or modified derivative thereof is provided. The modified derivative is preferably SEQ ID NO:35. Furthermore, for detection of the target nucleic acid sequence of SEQ ID NO: 36, a strand invasion oligonucleotide comprising SEQ ID NO 39 or a variant or modified derivative thereof is provided. The modified derivative is preferably SEQ ID NO:40. Additionally, for detection of the target nucleic acid sequence of SEQ ID NO: 41 a strand invasion oligonucleotide comprising SEQ ID NO 44 or a variant or modified derivative thereof is provided. The modified derivative is preferably SEQ ID NO:45. Finally, for detection of the target nucleic acid sequence of SEQ ID NO: 46 a strand invasion oligonucleotide comprising SEQ ID NO 49 or a variant or modified derivative thereof is provided. The modified derivative is preferably SEQ ID NO:50.

All of the above strand invasion oligonucleotides are typically oligonucleotides of greater than 30 nucleotides in length. It will further be understood that suitable modified derivatives and variants of the strand invasion oligonucleotides for the additional target sequences described above may be selected in accordance with the same detailed criteria described in relation to variants and modified derivatives of the strand invasion oligonucleotides for SEQ ID NOs 1 and 6.

A strand invasion oligonucleotide of the invention may further comprise a detectable label, for example a fluorescent dye.

### Amplification of the target nucleic acid sequence

The nucleic acid derived from the sample is contacted with the upstream and downstream primers and the strand invasion oligonucleotide for detection purposes, under conditions promoting amplification of the target nucleic acid sequence. Such conditions typically comprise the presence of a DNA polymerase enzyme. Suitable conditions include any conditions used to provide for activity of polymerase enzymes known in the art.

The conditions typically include the presence of all four dNTPs, dATP, dTTP, dCTP and dGTP or analogues thereof, suitable buffering agents/pH and other factors which are required for enzyme performance or stability. The conditions may include the presence of detergents and stabilising agents. The temperature used is typically isothermal, i.e constant throughout the amplification process. The temperature used typically depends on the nature of the polymerase enzyme and other enzyme components, and also reflects the hybridisation temperature required for the primers and strand invasion oligonucleotides. Where Bsu polymerase is used, a suitable temperature is 40 degrees centigrade.

The polymerase used typically has strand-displacement activity. The term "strand displacement" is used herein to describe the ability of a DNA polymerase, optionally in conjunction with accessory proteins, to displace complementary strands on encountering a region of double stranded DNA during DNA synthesis. Suitable DNA polymerases include poll from *E. coli, B. subtilis,* or *B. stearothermophilus,* and functional fragments or variants thereof, and T4 and T7 DNA polymerases and functional fragments or variants thereof. A preferred polymerase is Bsu DNA polymerase or a functional fragment or variant thereof.

The conditions may further comprise the presence of a recombinase. Any recombinase system may be used in the method of the invention. The recombinase system may be of prokaryotic or eukaryotic origin, and may be bacterial, yeast, phage, or mammalian. The recombinase may polymerise onto a single-stranded oligonucleotide in the 5'-3' or 3'-5; direction. The recombinase may be derived from a myoviridae phage, such as T4, T2, T6, Rb69, Aeh1, KVP40, Acinetobacter phage 133, Aeromonas phage 65, cyanophage P-SSM2, cyanophage PSSM4, cyanophage S-PM2, Rbl4, Rb32, Aeromonas phage 25, Vibrio phage nt-1, phi-1, Rbl6, Rb43, Phage 31, phage 44RR2.8t, Rb49, phage Rb3, or phage LZ2. In a preferred embodiment, the T4 recombinase UvsX (Accession number: P04529) or a functional variant or fragment thereof is used. The Rad systems of eukaryotes or the recA-Reco system of *E. coli* or other prokaryotic systems may also be used.

The conditions may further comprise the presence of recombinase accessory proteins, such as single-stranded binding protein (e.g. gp32, accession number P03695) and recombinase loading agent (e.g. UvsY, accession number NP_{_}049799.2). In a preferred embodiment, the conditions comprise the presence of the T4 gp32, UvsX and UvsY proteins.

The recombinase (such as UvsX), and where used the recombinase loading agent (such as UvsY) and single stranded DNA binding protein (such as gp32), can each be native, hybrid or mutant proteins from the same or different myoviridae phage sources. A native protein may be a wild type or natural variant of a protein.

The conditions may further comprise other factors used to enhance the efficiency of the recombinase such as compounds used to control DNA interactions, for example proline, DMSO or crowding agents which are known to enhance loading of recombinases onto DNA (Lavery P et. Al JBC 1992, 26713, 9307-9314; WO2008/035205).

The conditions may also comprise the presence of an ATP regeneration system. Various ATP regeneration systems are known to the person skilled in the art, and include glycolytic enzymes. Suitable components of an ATP regeneration system may include one or more of phosphocreatine, creatine kinase, myokinase, pyrophosphatase, sucrose and sucrose phosphorylase. The conditions may further comprise the presence of ATP.

Additional components such as magnesium ions, DTT or other reducing agents, salts, BSA/PEG or other crowding agents may also be included.

The various components described above, inclusive of the primers and strand invasion oligonucleotide, may be provided in varying concentrations to provide for DNA amplification. The skilled person can select suitable working concentrations of the various components in practice.

### Detection of presence of amplified DNA

The presence of amplified DNA resulting from the contacting of the target nucleic acid sequence with the primers and strand invasion oligonucleotide under conditions promoting DNA amplification may be monitored by any suitable means.

One or both of the primers, or the strand invasion oligonucleotide may incorporate a label or other detectable moiety. Any label or detectable moiety may be used. Examples of suitable labels include radioisotopes or fluorescent moieties, and FRET pairs of a fluorophore and acceptor moiety. Alternatively, or additionally one or more probes that detect the amplified DNA may be used, again incorporating a label or other detectable moiety. The probes may bind at any suitable location in the amplicon. Probes detecting different amplified target sequences may signal at different fluorescent wavelengths to provide for multiplex detection. The probe may be as described in WO 2015/075198. Thus, the probe may be an oligonucleotide comprising a fluorophore, a quencher and a region complementary to said target nucleic acid sequence, wherein the sequence of said oligonucleotide probe comprises at least 20% RNA nucleotides, modified RNA nucleotides and/or PNA nucleotides. Dyes which intercalate with amplified DNA may also be used to detect the amplified DNA, such as SYBR green and thiazole orange.

The detection of the signal from the amplified DNA may be made by any suitable system, including real-time PCR.

### Primers and oligonucleotides

The invention further provides each of the primers and strand invasion oligonucleotides of SEQ ID NOs 2 to 5,7 to 10, 12 to 15, 17 to 20, 22 to 25, 27 to 30; 32 to 35, 37 to 40, 42 to 45, 47 to 50 and variants and modified derivatives of each thereof as products per se, and compositions and formulations comprising said primers and strand invasion oligonucleotides. The primers and optionally the strand invasion oligonucleotide may be used in any method for detection of an influenza virus. Typically, the method is a strand-invasion based DNA amplification method. However, any suitable DNA amplification method that allows for specific detection of an influenza virus may be used. The upstream and downstream primers may be used in a DNA amplification method that does not require use of a strand invasion oligonucleotide, such as PCR.

### Combinations of primers and strand invasion oligonucleotides

It will be understood that the particular primers and strand invasion oligonucleotides (and variants/modified derivatives thereof) described for use in connection with particular target sequences herein are also described together for use in combination for amplification of that target sequence. Thus, for example, an upstream primer of less than 30 nucleotides in length comprising the sequence of SEQ ID NO:12 or a variant thereof, a downstream primer of less than 30 nucleotides in length comprising the sequence of SEQ ID NO:13 or a variant thereof, and a strand invasion oligonucleotide of greater than 30 nucleotides in length comprising the sequence of SEQ ID NO:14 or a variant or modified derivative thereof (typically comprising one or more modified nucleotides in its 3' region), are described for use in methods for amplification of the target sequence of SEQ ID NO: 11 or a variant thereof, and also for inclusion in combination in kits and compositions. The same principles apply to the other primer/strand invasion oligonucleotides described for each target sequence herein.

### Compositions and kits

The invention also provides compositions and kits comprising at least two oligonucleotides selected from (a) an upstream primer, (b) a downstream primer and (c) a strand invasion oligonucleotide. The upstream primer, downstream primer and strand invasion oligonucleotide are as described above. The composition or kit may comprise an upstream and a downstream primer, an upstream primer and a strand invasion oligonucleotide, or a downstream primer and a strand invasion oligonucleotide. Preferably, the composition or kit comprises an upstream primer, a downstream primer and a strand invasion oligonucleotide. The composition or kit may be suitable for detection of an influenza virus, in accordance with the method of the invention, or an alternative DNA amplification method.

Where the composition or kit is suitable for use for detecting the target nucleic acid sequence of SEQ ID NO: 1, typically the upstream primer is an oligonucleotide of less than 30 nucleotides in length comprising the sequence of SEQ ID NO: 2 or a variant thereof, the downstream primer is an oligonucleotide of less than 30 nucleotides in length comprising the sequence of SEQ ID NO: 3 or a variant thereof, and the strand invasion oligonucleotide is an oligonucleotide of greater than 30 nucleotides in length comprising the sequence of SEQ ID NO: 4 or a variant thereof, and further comprising one or more modified nucleotides in its 3'region. The strand invasion oligonucleotide may have the sequence of SEQ ID NO: 5.

Where the composition or kit is suitable for use for detecting the target nucleic acid sequence of SEQ ID NO: 6, typically the upstream primer is an oligonucleotide of less than 30 nucleotides in length comprising the sequence of SEQ ID NO: 7 or a variant thereof, the downstream primer is an oligonucleotide of less than 30 nucleotides in length comprising the sequence of SEQ ID NO: 8 or a variant thereof, and the strand invasion oligonucleotide is an oligonucleotide of greater than 30 nucleotides in length comprising the sequence of SEQ ID NO: 9 or a variant thereof, and further comprising one or more modified nucleotides in its 3'region. The strand invasion oligonucleotide may have the sequence of SEQ ID NO: 10.

The composition or kit may provide a first set of oligonucleotides allowing for detection of the target nucleic acid sequence of SEQ ID NO: 1 and additionally a second set of oligonucleotides allowing for detection of the target nucleic acid sequence of SEQ ID NO: 6.

The combinations of upstream primer, downstream and strand invasion oligonucleotide described above for the target sequences of SEQ ID NOs 11, 16, 21, 26, 31, 36, 41 and 46 may also be selected to provide compositions and kits in a similar manner to that described above.

The above composition may be for example a solution, lyophilisate, suspension, or an emulsion in an oily or aqueous vehicle.

In the above kit, the at least two oligonucleotides may be provided as a mixture, or in separate containers. The kit optionally further comprises instructions for use in a method of the invention. The kit may comprise a means for detection of amplified DNA.

The kit or composition optionally comprises one or more probes that detect amplified DNA. The kit or composition optionally comprises one or more of a DNA polymerase, a reverse transcriptase, a recombinase, and recombinase accessory proteins. The kit may comprise both a reverse transcriptase and a DNA polymerase, or both a reverse transcriptase and a recombinase. Preferably, the DNA polymerase is Bsu polymerase. Preferably, the recombinase is bacteriophage T4 UvsX, optionally in combination with the recombinase accessory proteins UvsY and gp32. The kit or composition may further comprise dNTPs, suitable buffers and other factors which are required for DNA amplification in the method of the invention as described above.

### Diagnosis of an infection by a pathogen and medical applications

The present invention is particularly advantageous in the medical setting. The detection methods of the invention provide a highly specific test to allow for determination of whether a clinical sample contains a target nucleic acid sequence from a pathogen, particularly an influenza virus. The method may be applied to a range of disease settings associated with influenza virus. Additionally, the method may be applied for screening of carriers of influenza virus.

The determination of whether or not influenza virus is present may be in the context of any disease or illness present or suspected of being present in a patient. Such diseases may include those caused by, linked to, or exacerbated by the presence of influenza virus. Thus, a patient may display symptoms indicating the presence of influenza virus, such as a respiratory illness, and a sample may be obtained from the patient in order to determine the presence of influenza virus and optionally also the serotype, subtype or strain thereof by the method described above.

The invention thus provides a method of diagnosing an infection caused by influenza virus in a subject, comprising determining the presence of a target nucleic acid sequence from influenza virus according to the invention in a sample from said subject. The method may further comprise other steps of identifying the strain of influenza virus, such as by serology, immunoassay or viral culture from a sample provided by the subject.

A particularly preferred embodiment of the invention is the identification of influenza virus present in patients having a respiratory illness.

The invention thus provides a diagnostic method for respiratory illnesses that are caused by influenza virus. The method provides for a dramatic improvement in the patient management of respiratory illnesses because it allows for the optimal therapeutic treatment for a given patient. Thereby the test would reduce the length of hospital stays, the frequency of re-admission and reduce costs. The amplification method of the invention has particular benefits over other nucleic acid amplification methods in the clinical setting. RT-PCR requires the use of heavy and sophisticated thermal cyclers and skilled personnel, which consequently limits its use for in field or point-of-care applications. The amplification method of the invention can be carried out isothermally only requiring small instruments, enabling it to be performed within point-of-care settings. This provides benefits for prompt treatment of patients and limitation of the misuse of antibiotics or antiviral drugs by early identification of the infecting pathogen associated with a respiratory illness.

Influenza A and influenza B are among the most common cause of acute human respiratory illness with high morbidity and mortality rates in infants, elderly and immunocompromised individuals [Mallia et al. 2007, Simonsen et al. 2000]. Influenza vaccination is the most effective strategy for preventing the spread of the infection and as such vaccines are often recommended to individuals with high risk of complications [Flannery et al. 2015]. However, due to the variability amongst influenza strains and subtypes, a single vaccine is not adequate in protecting against all influenza strains and subtypes. Consequently multiple vaccines may need to be administered to patients during every influenza season or during the emergence of a new strain. Patients with influenza infection may also be administered with antiviral drugs. However these antiviral drugs are only effective within the first two days of the illness [Stiver 2003]. Early and effective diagnosis of influenza consequently plays an important role in the treatment and management of the illness. Furthermore, it plays an importance role in prevention of antibiotics misuse since influenza symptoms are often similar to respiratory illnesses caused by bacterial infections [Low 2008].

The diagnostic method may conveniently be performed based on nucleic acid derived from a sample of a patient, providing an indication to clinicians whether the respiratory illness is due to an infection by influenza virus. The diagnostic method may also provide an indication as to the serotype, strain or subtype of influenza and it can then be evaluated whether this is known to be resistant to any antiviral compounds. Depending on the outcome of the test the medical treatment can then be optimised, for example by use of appropriate antiviral compounds.

The following Examples illustrate the invention.

### Example 1 - Selection of target sequences and oligonucleotides for detection of Influenza A and B virus using RT-SIBA

The isothermal nucleic acid amplification method known as Strand Invasion Based Amplification (SIBA®) with high analytical sensitivity and specificity has previously been described in Hoser et al.

An assay incorporating the SIBA method, namely reverse transcription SIBA (RT-SIBA) was developed for rapid detection of viral RNA targets. The RT-SIBA method includes a reverse transcriptase enzyme that allows a one-step reverse transcription of RNA to cDNA and simultaneous amplification and detection of cDNA with SIBA.

The Influenza Research Database (http://www.fludb.org) was utilized in the design of influenza RT-SIBA assays (Squires et al) for detection of Influenza and B. In order to design an assay for influenza A altogether 4575 non-duplicate Influenza A segment 1 sequences that had been submitted during years 2010-2015 were retrieved and aligned. Special emphasis was placed on the clinically most relevant subtypes H1N1, H3N2 and H5N1 and, thus, sequences representing these subtypes were included in the alignment.

The alignment was analysed to find areas of limited variability using Jalview (http://www.jalview.org). In these analyses, a highly conserved area of around 80 nucleotides in length was found in the 3' end of the aligned segment 3 with a suitable consensus sequence (SEQ ID NO:51; see Figure 1) and this area was selected as one target for RT-SIBA assay design.

Analogously, for influenza B assays, an alignment of 1495 non-duplicate segment 3 sequences was produced. A highly conserved area of around 80 nucleotides in length was identified right at the beginning of the alignment (SEQ ID NO:52; see Figure 1) and selected as one assay target area.

SIBA amplification is highly specific, and thus, special care was taken to ensure that most of the sequence variation would fall inside the forward or reverse oligonucleotide sequences and preferably only to a limited degree, i.e. maximally 1-2 changes, in the invasion oligonucleotide area. Hoser et al. provides general information on general assay design, SIBA specificity and initial screening of suitable oligonucleotide combinations.

After being established, the assays were then subjected to several rounds of optimization, as described subsequently. The preferred target region (amplicon) for detecting Influenza A based on the consensus region of SEQ ID NO: 51 discussed above was established as SEQ ID NO:1 (IFAX) and preferred primers and strand invasion oligonucleotides were designed for amplification of this target. The preferred target region (amplicon) for detecting Influenza B based on the consensus region of SEQ ID NO: 52 discussed above was established as SEQ ID NO:6 (IFB4) and preferred primers and strand invasion oligonucleotides were designed for amplification of this target.

### Example 2 - RT-SIBA method and optimisation of oligonucleotide design

RT-SIBA reactions described herein were performed using a commercial SIBA reagent kit (Orion Diagnostica Oy, Finland) with the addition of 16U of GoScript™ Reverse Transcriptase (Promega). UvsX and gp32 enzymes were each used at 0.25 mg/ml and the reaction were started using 10 mM magnesium acetate. The forward and reverse primers and the IO were each used at 200 nM final concentrations. The oligonucleotides used were purchased from Eurofins Genomics (Ebersberg, Germany) or Integrated DNA Technologies (Leuven, Belgium) and purified by the manufacturer using either reverse-phase HPLC (for primers and probes) or PAGE (for the invasion oligonucleotides).

SIBA amplification was detected using SYBR Green 1 (1:100,000 dilution). RT-SIBA reactions were incubated at 41°C for 60 minutes, and fluorescence readings were taken at 60-second intervals on an Agilent MX Pro 3005P (Agilent Technologies, Inc., CA, United States). After incubation for 60 minutes, the instrument was set to run a melt curve from 40°C to 95°C in order to further assess the specificity of SIBA reactions.

SIBA reactions were optimized for the rapid detection of influenza A by first determining the best nucleotide configuration of invasion oligonucleotide (IO) that gives the shortest detection time. For this amplification assay, a 300 bp template sequence harboring region 2023-2322 from Influenza A virus segment 1 polymerase PB2 gene (KJ942711) was cloned into pEXA2 vector (Eurofins, Germany). The sequence was flanked by a 20 bp T7 promoter sequence and a SmaI site at the 5' and 3' end respectively. Transcripts were prepared using an in vitro transcription kit, namely HiScribe™ T7 High Yield RNA Synthesis Kit (New England Biolabs, Ipswich, USA). Transcripts were quantified both spectrophotometrically and by quantitative real-time polymerase chain reaction.

RNA transcripts were then used to determine the performance of an influenza A RT-SIBA assay in the context of strand invasion oligonucleotides having different nucleotide configurations. This was done by varying the length and composition of the 5' non-homologous (seeding) region of the IO. Six different invasion oligonucleotides (IO) having lengths of the seeding region varying from 0 to 20 nucleotides were tested.

In another approach, the length of the seeding region was kept at 14 nucleotides but the nucleotide composition of the seeding region was modified to contain either polyC, polyT, polyA, polyG, poly purines or poly pyrimidines.

The performance of each IO was assessed by its ability to facilitate amplification of 10,000 copies of *in vitro* transcribed influenza A RNA in the presence of the primers.

The results are depicted in Figure 2. Our results showed that the reactions with IO containing no seeding region displayed the longest detection time suggesting that the seeding region is of vital importance for efficient amplification of the target DNA. Furthermore, shorter detection times were observed as the length of the seeding region increased. Our experiments demonstrated that the optimal length of the seeding region in terms of assay speed was between 14 and 20 nucleotides.

To further evaluate the impact of the seeding region nucleotide composition on assay speed six IOs with seeding regions either containing polyC, polyT, polyA, polyG, or mixtures of poly purines or poly pyrimidines were tested. Of these, the IOs containing a seeding region with polyC displayed the shortest detection time while IOs containing polyG seeding region failed to amplify the transcript at all. IOs containing a seeding region with a mixture of pyrimidine nucleotides was found to display shorter detection times than that with a mixture of purine nucleotides.

In conclusion, IOs for influenza A and B assays that were used in the following experiments were preferably designed to contain seeding regions that consisted of a polyC with 14 nucleotides in length.

### Example 3 - Comparison of RT-SIBA with RT-PCR

The performance of RT-SIBA for the detection of influenza A and B were compared with the Centers for Disease Control and Prevention (CDC) protocol for real time RT-PCR for Influenza A and B, as described in the Selvariu and WHO references cited below.

The RT-SIBA reactions were performed as outlined in Example 2, using the primers and strand invasion oligonucleotides of SEQ ID NOs 2, 3 and 5 for detection of Influenza A, and the primers and strand invasion oligonucleotides of SEQ ID NOs 7, 8 and 10 for detection of Influenza B. Briefly, the RT-PCR reactions were performed with EXPRESS One-Step SuperScript® qRT-PCR SuperMix Kit ROX Reference Dye (Thermo Fisher Scientific USA),). The EXPRESS One-Step SuperScript® qRT-PCR SuperMix Kit also contains a heat-labile form of uracil DNA glycosylase (UDG) in addition to the SuperScript® III Reverse Transcriptase and Platinum® Taq DNA polymerase in order to prevent any potential carry-over contamination. The reactions were detected with an Applied Biosystem 7500 PCR instrument (Thermo Fisher Scientific USA). All primers and probes were used at 1000 nM and 200 nM respectively. The following thermal cycling protocol was used: 50°C for 30 min (cDNA synthesis), 95°C for 2 min (reverse transcriptase and UDG inactivation), and 45 cycles of 95°C for 15 s and 55°C for 30 s (PCR amplification).

### Improved sensitivity and inclusivity of Influenza A and B RT-SIBA assays in comparison to RT-PCR

Sensitivity of both influenza A and B RT-SIBA assays were assessed against the previously published CDC real-time RT-PCR assays. Amplirun purified and quantified RNA for influenza A (H1N1, H3N2 and H5N1) and influenza B were obtained from Vircell (Granada, Spain) and were used to determine the analytical sensitivity of both RT-SIBA and RT-qPCR methods for influenza A and B. Serial dilutions of quantified Amplirun RNA controls for influenza A H1N1, H3N2 and H5N1 subtypes as well as influenza B were used as templates for both RT-SIBA and RT-PCR runs. Quadruplicate reactions were run in at least three independent experiments. The diluted samples were analysed simultaneously with both RT-SIBA and RT-PCR reactions in order to facilitate comparison between the two methods. The RT-SIBA and RT-PCR methods were as described above. The primers used in each method for detection of influenza A were SEQ ID NOs 2 and 3, and for RT-SIBA additionally the strand invasion oligonucleotide of SEQ ID NO:5 was used. The primers used in each method for detection of influenza B were SEQ ID NOs 7 and 8, and for RT-SIBA additionally the strand invasion oligonucleotide of SEQ ID NO:9 was used. The results are shown in Figure 3 (RT-SIBA data only) and Table 1 (Summary of RT-SIBA and RT-PCR data).

RT-SIBA reliably detected as low as 10 copies of viral RNA per reaction. Serial dilutions from 1000 to 10 copies of purified RNA from different influenza subtypes showed reproducible and specific amplification when detected with Sybr green I fluorescent dye. Further, post-amplification melt curve analysis was performed after each run. Notably, amplification of a single specific amplicon was detected with both fluA and fluB assays (data not shown). Furthermore, influenza A RT-SIBA assay did not cross-react with influenza B RNA and neither did influenza B assay cross-react with influenza A RNA.

Comparison of analytical sensitivity in terms of RNA copy numbers per reaction indicated that RT-SIBA assays had at least comparable or superior sensitivity compared to RT-PCR assays. The results are shown in Table 1. Notably, we were able to demonstrate sensitivity of RT-SIBA to be 10 cp of target RNA per reaction for both IFA1 and IFB4 assays. However, our experiments demonstrated that as opposed to the RT-SIBA assays the sensitivity of RT-PCR varied from 10 to 1000 cp per reaction depending on the assay and influenza template subtype.

In case of influenza B assays RT-SIBA and RT-PCR assays performed equally well and were able to successfully amplify 10 cp of RNA per reaction. However, significant difference between RT-SIBA and RT-PCR was observed with influenza A assays. 10 copies of all tested subtypes of influenza A were reproducibly amplified and detected by RT-SIBA whereas RT-PCR assays were only capable of amplifying 100 or 1000 cp or RNA per reaction.

Average time for positive reaction that corresponded to the Ct-values of RT-PCR reactions was calculated according to the one-step RT-PCR cycling programme without taking into account ramping time needed for PCR cycling. RT-SIBA reactions were performed at constant temperature and data was collected in 1 min intervals. Therefore, the Ct-values of RT-SIBA reactions corresponded directly to the time for positive result. The average times for positive results of both RT-SIBA and RT-PCR reactions are listed in Table 1.

Positive result for 100 cp influenza B RNA was obtained in average in 12 min with RT-SIBA whereas in average 53 min was needed to yield a positive result with RT-PCR. Similarly, 100 cp of influenza A H1N1, H3N2 and H5N1 subtypes were detected with RT-SIBA in 26 min, 23 min and 21 min, respectively, whereas more than 50 min reaction time was needed for RT-PCR. This demonstrated a major advantage of RT-SIBA over RT-PCR.

**Table 1. Sensitivity of FluA and FluA RT-SIBA assays compared to CDC RT-PCR assays.**

| | | No of positive reactions (average time of positive result*) | | | |
|---|---|---|---|---|---|
| | | RT-SIBA | | RT-PCR | |
| Subtype | cp/reaction | FluA | FluB | CDC-A | CDC-B |
| A (H1N1) | 1000 | 12/12 (12 min) | | 12/12 (54 min) | |
| | 100 | 12/12 (15 min) | | 0/12 | |
| | 10 | 12/12 (20 min) | | 0/12 | |
| A (H3N2) | 1000 | 12/12 (11 min) | | 12/12 (50 min) | |
| | 100 | 12/12 (14 min) | | 12/12 (52 min) | |
| | 10 | 12/12 (16 min) | | 0/12 | |
| A (H5N1) | 1000 | 12/12 (10 min) | | 12/12 (51 min) | |
| | 100 | 12/12 (12 min) | | 12/12 (53 min) | |
| | 10 | 12/12 (15 min) | | 0/12 | |
| B | 1000 | | 12/12(10 min) | | 12/12 (51 min) |
| | 100 | | 12/12 (12 min) | | 12/12 (53 min) |
| | 10 | | 11/12(13 min) | | 11/12 (56 min) |

| | | | | | |
|---|---|---|---|---|---|
| *ramp time of RT-PCR reactions not included in the calculations | | | | | |

In summary, the average time to detection for both influenza A and B RT-SIBA assays were below 15 minutes for 1000 copies of RNA. Thus, the total test can be conducted in less than 30 minutes. This was significantly faster than the real-time RT-PCR method which took over one hour for the detection of influenza. In RT-SIBA, the reverse transcription and the cDNA amplification occur simultaneously in the same reaction temperature, which, thus, allow for rapid detection of target RNA. In contrast, in real-time RT-PCR the reverse transcription required an initial incubation period of 30 minutes at a constant temperature prior to the repeated cycles of denaturation, annealing and elongation. That resulted in a total run time of between 90-120 minutes. Furthermore, RT-SIBA was found to be more sensitive than RT-PCR for the detection of influenza A and B. RT-SIBA displayed a 100 fold sensitivity compared to RT-PCR for the detection for H1N1.

### Example 4 - Detection of other Influenza A and B target regions with RT-SIBA

Eight further consensus sequences for detection of Influenza A and B viruses were identified in various gene segments based on the approach outlined in Example 1. Preferred amplicon regions and primers/strand invasion oligonucleotides for their amplification were then selected, and used for RT-SIBA detection of Influenza A or B in assays as described in Example 2. In summary:
- The IFA1 amplicon (SEQ ID NO: 11) was identified within the consensus sequence of SEQ ID NO 53 from segment 1 of Influenza A. The primers and strand invasion oligonucleotides of SEQ ID NOs 12-13 and 15 were used for amplification of SEQ ID NO:11.
- The INFLA3 amplicon (SEQ ID NO: 16) was identified within the consensus sequence of SEQ ID NO 54 from segment 3 of Influenza A. The primers and strand invasion oligonucleotides of SEQ ID NOs 17-18 and 20 were used for amplification of SEQ ID NO:16.
- The IFA2 amplicon (SEQ ID NO: 21) was identified within the consensus sequence of SEQ ID NO 55 from segment 7 of Influenza A. The primers and strand invasion oligonucleotides of SEQ ID NOs 22-23 and 25 were used for amplification of SEQ ID NO:21.
- The IFA4 amplicon (SEQ ID NO: 26) was identified within the consensus sequence of SEQ ID NO 56 from segment 1 of Influenza A. The primers and strand invasion oligonucleotides of SEQ ID NOs 27-28 and 30 were used for amplification of SEQ ID NO:26.
- The IFA5 amplicon (SEQ ID NO: 31) was identified within the consensus sequence of SEQ ID NO 57 from segment 1 of Influenza A. The primers and strand invasion oligonucleotides of SEQ ID NOs 32-33 and 35 were used for amplification of SEQ ID NO:31.
- The IFAbl amplicon (SEQ ID NO: 36) was identified within the consensus sequence of SEQ ID NO 58 from segment 3 of Influenza A. The primers and strand invasion oligonucleotides of SEQ ID NOs 37-38 and 40 were used for amplification of SEQ ID NO:36.
- The IFAbl4 amplicon (SEQ ID NO: 41) was identified within the consensus sequence of SEQ ID NO 59 from segment 3 of Influenza A. The primers and strand invasion oligonucleotides of SEQ ID NOs 42-43 and 45 were used for amplification of SEQ ID NO:41.
- The IFB2/3 amplicon (SEQ ID NO: 46) was identified within the consensus sequence of SEQ ID NO 60 from segment 3 of Influenza B. The primers and strand invasion oligonucleotides of SEQ ID NOs 47-48 and 50 were used for amplification of SEQ ID NO:46.
Results of the assays for detection the additional target regions are shown in Figures 4-8. Differing subtypes of Influenza A were used for detection in some instances as shown, and also sensitivity was only determined at one or two RNA copy numbers. A full determination of sensitivity and inclusivity was thus not performed for the additional target regions, but in each case they were found to be suitable for detection of one or more Influenza subtypes with good sensitivity.

Accordingly, RT-SIBA was found to be a particularly suitable method for detection of Influenza A or B based on a variety of different target regions and gene segments of the viruses.

### Example 5 - Analytical specificity of SIBA influenza A and B and clinical panel performance

The performance of the two SIBA flu A and B assays described in Examples 2 and 3 was further evaluated using a commercial available Influenza Verification Panel that contains influenza viral particles in proteins that simulates clinical sample matrix (NATrol Flu Verification Panel; ZeptoMetrix Buffalo, NY). The panel contained six influenza A virus strains from H1 and H3, two Influenza B strains, Respiratory Syncytial Virus A, Respiratory Syncytial Virus B, Rhinovirus 1A, Parainfluenza virus Type 1, Echovirus Type 30, Coxsackievirus type A9, M. pneumoniae, strain M129 and N. meningitides Serogroup A. Each pathogen was quickly lysed with 2% Triton X 100 and added to the reaction.

Results are shown in Figures 9A-B (IFAX) and 10A (IFB4), and Table 2 below. The SIBA fluA assay detected all six influenza A strains and did not cross react with influenza B strains nor the other respiratory pathogens. Similarly, the SIBA influenza B assay detected the two Influenza B strains and did not cross react with influenza A strains nor the other respiratory pathogens. This suggests that SIBA influenza A and B assays could be suitable for detecting influenza viruses from clinical specimens.

**Table 2. NATrol Flu verification panel test results.**

| **Panel Member** | **Strain** | **SIBA Result, IFAX** | **SIBA Result, IFB4** |
|---|---|---|---|
| Influenza A H1N1 | A/NewCaledonia/20/99 | + | **-** |
| Influenza A H1N1 | A/Brisbane/59/07 | + | - |
| Influenza A H3N2 | A/Brisbane/10/07 | + | - |
| Influenza A H3N2 | A/Wisconsin/67/05 | + | - |
| Influenza A 2009 H1N1 | Canada/6294/09 | + | - |
| Influenza A 2009 H1N1 | NY/02/09 | + | - |
| Influenza B | B/Florida/02/06 | - | + |
| Influenza B | B/Malaysia/2506/04 | - | + |
| Respiratory Syncytial Virus A | NA | - | - |
| Respiratory Syncytial Virus B | CH93(18)-18 | - | - |
| Rhinovirus 1A | NA | - | - |
| Parainfluenza virus Type 1 | NA | - | - |
| Echovirus Type 30 | NA | - | - |
| Coxsackievirus type A9 | NA | - | - |
| *M. pneumoniae* | M129 | - | - |
| *N. meningitidis* Serogroup A | NA | - | - |

As an additional demonstration of detection in a clinical setting, an IFAX assay as described in Examples 2 and 3 was carried out in the presence or absence of UTM (universal transport medium). UTM is typically used as a buffer for storage of clinical samples taken by nose/throat swabs. Results are shown in Figure 10B. As expected from the general effects of UTM on nucleic acid amplification assays, there was a slight inhibition of sensitivity in the presence of UTM. However, the reaction was still highly sensitive in the presence of UTM, illustrating the suitability of RT-SIBA for detection from clinical samples.

The performance of RT-SIBA for the detection of influenza A and B was thus shown to be suitable with commercially available clinical flu verification panel samples that simulate the clinical sample specimen. RT-SIBA influenza assays amplified specifically the influenza specimen without cross-reacting with other respiratory pathogens. Since RT-SIBA was found to be less susceptible to sample derived inhibition, the method consequently does not require highly purified RNA as template. Crude disruption of the viral particle by either heating or detergents was found to be sufficient. The sensitive and rapid detection within 15 mins as well as the tolerance to sample derived inhibition prove these assays to be a powerful molecular diagnostics tool. Since the method is run at a low and constant temperature potentially battery operated and portable devices of considerably lower complexity than what would be used in RT-qPCR can be employed. The methods of the invention can thus be suitable outside the centralized laboratory space in lower complexity settings like smaller laboratories, in the field or even in home use.

### References

Drosten C, Panning M, Guenther S, Schmitz H: False-Negative Results of PCR Assay with Plasma of Patients with Severe Viral Hemorrhagic Fever. Journal of Clinical Microbiology 2002, 40(11):4394-4395.
Flannery B, Clippard J, Zimmerman RK, Nowalk MP, Jackson ML, Jackson LA, Monto AS, Petrie JG, McLean HQ, Belongia EA et al: Early estimates of seasonal influenza vaccine effectiveness - United States, January 2015. MMWR Morb Mortal Wkly Rep 2015, 64(1):10-15.
Hoser M, Mansukoski HK, Morrical SW and Eboigbodin KE: Strand Invasion Based Amplification (SIBA): A Novel Isothermal DNA Amplification Technology Demonstrating High Specificity and Sensitivity for a Single Molecule of Target Analyte. PLoS One 2014, 9(11): e112656.
Low D: Reducing antibiotic use in influenza: challenges and rewards. Clinical Microbiology and Infection 2008, 14(4):298-306.
Mahony JB, Petrich A, Smieja M: Molecular diagnosis of respiratory virus infections. Critical Reviews in Clinical Laboratory Sciences 2011, 48(5-6):217-249.
Mallia P, Johnston SL: Influenza infection and COPD. International Journal of Chronic Obstructive Pulmonary Disease 2007, 2(1):55-64.
Selvaraju SB, Selvarangan R: Evaluation of Three Influenza A and B Real-Time Reverse Transcription-PCR Assays and a New 2009 H1N1 Assay for Detection of Influenza Viruses. Journal of Clinical Microbiology 2010, 48(11):3870-3875.
Simonsen L, Fukuda K, Schonberger LB, Cox NJ: The Impact of Influenza Epidemics on Hospitalizations. Journal of Infectious Diseases 2000, 181(3):831-837.
Squires RB, Noronha J, Hunt V, Garcia-Sastre A, Macken C, Baumgarth N, Suarez D, Pickett BE, Zhang Y, Larsen CN et al: Influenza Research Database: an integrated bioinformatics resource for influenza research and surveillance. Influenza and Other Respiratory Viruses 2012, 6(6):404-416.
Stiver G: The treatment of influenza with antiviral drugs. CMAJ: Canadian Medical Association Journal 2003, 168(1):49-57.
(WHO) WHO: CDC protocol of real-time RT-PCR for influenza H1N1. World Health Organization, Geneva, Switzerland. In., vol. http://www.who.int/csr/resources/publications/swineflu/realtimeptpcr/en/index.html; 30 April 2009.

## Claims

1. A method for detecting a ribonucleic acid (RNA) comprising a target nucleic acid sequence from an Influenza virus in a sample, said method comprising contacting said sample with a reverse transcriptase, at least one upstream primer, at least one downstream primer and at least one strand invasion oligonucleotide under conditions promoting amplification of said target nucleic acid sequence,
wherein each said primer and said strand invasion oligonucleotide comprises a region complementary to said target nucleic acid sequence;
wherein said strand invasion oligonucleotide renders at least a portion of a duplex nucleic acid comprising said target nucleic acid sequence single-stranded, to allow the binding of said upstream primer and a downstream primer;
wherein:
(i) the amplified target nucleic acid sequence comprises SEQ ID NO: 1, and the upstream primer comprises SEQ ID NO: 2 or a variant thereof and the downstream primer comprises SEQ ID NO: 3 or a variant thereof and the strand invasion oligonucleotide comprises SEQ ID NO: 4 or a variant thereof;
(ii) the amplified target nucleic acid sequence comprises SEQ ID NO: 6, and the upstream primer comprises SEQ ID NO: 7 or a variant thereof and the downstream primer comprises SEQ ID NO: 8 or a variant thereof and the strand invasion oligonucleotide comprises SEQ ID NO: 9 or a variant thereof;
(iii) the amplified target nucleic acid sequence comprises SEQ ID NO: 11, and the upstream primer comprises SEQ ID NO: 12 or a variant thereof and the downstream primer comprises SEQ ID NO: 13 or a variant thereof and the strand invasion oligonucleotide comprises SEQ ID NO: 14 or a variant thereof;
(iv) the amplified target nucleic acid sequence comprises SEQ ID NO: 16, and the upstream primer comprises SEQ ID NO: 17 or a variant thereof and the downstream primer comprises SEQ ID NO: 18 or a variant thereof and the strand invasion oligonucleotide comprises SEQ ID NO: 19 or a variant thereof;
(v) the amplified target nucleic acid sequence comprises SEQ ID NO: 21, and the upstream primer comprises SEQ ID NO: 22 or a variant thereof and the downstream primer comprises SEQ ID NO: 23 or a variant thereof and the strand invasion oligonucleotide comprises SEQ ID NO: 24 or a variant thereof;
(vi) the amplified target nucleic acid sequence comprises SEQ ID NO: 26, and the upstream primer comprises SEQ ID NO: 27 or a variant thereof and the downstream primer comprises SEQ ID NO: 28 or a variant thereof and the strand invasion oligonucleotide comprises SEQ ID NO: 29 or a variant thereof;
(vii) the amplified target nucleic acid sequence comprises SEQ ID NO: 31, and the upstream primer comprises SEQ ID NO: 32 or a variant thereof and the downstream primer comprises SEQ ID NO: 33 or a variant thereof and the strand invasion oligonucleotide comprises SEQ ID NO: 34 or a variant thereof;
(viii) the amplified target nucleic acid sequence comprises SEQ ID NO: 36, and the upstream primer comprises SEQ ID NO: 37 or a variant thereof and the downstream primer comprises SEQ ID NO: 38 or a variant thereof and the strand invasion oligonucleotide comprises SEQ ID NO: 39 or a variant thereof;
(ix) the amplified target nucleic acid sequence comprises SEQ ID NO: 41, and the upstream primer comprises SEQ ID NO: 42 or a variant thereof and the downstream primer comprises SEQ ID NO: 43 or a variant thereof and the strand invasion oligonucleotide comprises SEQ ID NO: 44 or a variant thereof; or
(x) the amplified target nucleic acid sequence comprises SEQ ID NO: 46, and the upstream primer comprises SEQ ID NO: 47 or a variant thereof and the downstream primer comprises SEQ ID NO: 48 or a variant thereof and the strand invasion oligonucleotide comprises SEQ ID NO: 49 or a variant thereof;
further wherein each said primer is an oligonucleotide of less than 30 nucleotides in length and wherein each strand invasion oligonucleotide is an oligonucleotide of more than 30 nucleotides in length, and wherein a said variant of a primer has at least 70% sequence identity to the sequence of the corresponding original sequence, and wherein a said variant of a strand invasion oligonucleotide comprises a region complementary to said target nucleic acid sequence having at least 70% sequence identity to the region complementary to said target nucleic acid sequence of the original strand invasion oligonucleotide, or comprises a complementary region to the region complementary to said target nucleic acid sequence of the original strand invasion oligonucleotide.

2. A method according to claim 1, wherein the strand invasion oligonucleotide comprises one or more modified nucleotides in its 3'region.

3. A method according to claim 1 or 2, wherein the variant of the primer sequences comprises a sequence having 1, 2, 3, or 4 mismatches to the sequence of the original primer sequence and the variant of the strand invasion oligonucleotide comprises a region complementary to the target nucleic acid sequence having 1, 2, 3, or 4 mismatches with respect to the corresponding region in the original strand invasion oligonucleotide sequence.

4. A method according to claim 1 or 2, wherein:
(i) the amplified target nucleic acid sequence comprises SEQ ID NO: 1 and the strand invasion oligonucleotide is SEQ ID NO: 5;
(ii) the amplified target nucleic acid sequence comprises SEQ ID NO: 6 and the strand invasion oligonucleotide is SEQ ID NO: 10;
(iii) the amplified target nucleic acid sequence comprises SEQ ID NO: 11 and the strand invasion oligonucleotide is SEQ ID NO: 15;
(iv) the amplified target nucleic acid sequence comprises SEQ ID NO: 16 and the strand invasion oligonucleotide is SEQ ID NO: 20;
(v) the amplified target nucleic acid sequence comprises SEQ ID NO: 21 and the strand invasion oligonucleotide is SEQ ID NO: 25;
(vi) the amplified target nucleic acid sequence comprises SEQ ID NO: 26 and the strand invasion oligonucleotide is SEQ ID NO: 30;
(vii) the amplified target nucleic acid sequence comprises SEQ ID NO: 31 and the strand invasion oligonucleotide is SEQ ID NO: 35;
(viii) the amplified target nucleic acid sequence comprises SEQ ID NO: 36 and the strand invasion oligonucleotide is SEQ ID NO: 40;
(ix) the amplified target nucleic acid sequence comprises SEQ ID NO: 41 and the strand invasion oligonucleotide is SEQ ID NO: 45;
(x) the amplified target nucleic acid sequence comprises SEQ ID NO: 46 and the strand invasion oligonucleotide is SEQ ID NO: 50;

5. A method according to any one of the preceding claims, which further comprises contacting of said sample with a recombinase.

6. A method according to any one of the preceding claims, wherein the strand invasion oligonucleotide has a 5' terminal region non-complementary to the target nucleic acid sequence which is at least about 14 nucleotides in length and/or is comprised of purine nucleotides preferably consisting essentially of cytosine nucleotides.

7. A composition comprising (a) an upstream primer, (b) a downstream primer and (c) a strand invasion oligonucleotide,
wherein each said oligonucleotide is as defined in any one of claims 1(i) to 1(x).

8. A kit comprising (a) an upstream primer, (b) a downstream primer and (c) a strand invasion oligonucleotide,
wherein each said oligonucleotide is as defined in any one of claims 1(i) to 1(x).

9. A composition or kit according to claim 7 or 8, further comprising
(i) a reverse transcriptase; and/or
(ii) a DNA polymerase; and/or
(iii) a recombinase.

10. A method for diagnosis of an infection by a pathogen in a subject, comprising carrying out a method as defined in any one of claims 1 to 6 in a sample from said subject.

## Patentansprüche

1. Verfahren zum Nachweis einer Ribonukleinsäure (RNA) umfassend eine Zielnukleinsäuresequenz aus einem Influenzavirus in einer Probe, das Verfahren umfassend Inkontaktbringen der Probe mit einer reversen Transkriptase, mindestens einem stromaufwärtigen Primer, mindestens einem stromabwärtigen Primer und mindestens einem Stranginvasionsoligonukleotid unter Bedingungen, die die Amplifizierung der Zielnukleinsäuresequenz fördern,
wobei jedes von dem Primer und dem Stranginvasionsoligonukleotid eine zur Zielnukleinsäuresequenz komplementäre Region umfasst;
wobei das Stranginvasionsoligonukleotid mindestens einen Teil einer Duplex-Zielnukleinsäuresequenz umfassend die Zielnukleinsäuresequenz einsträngig macht, um die Bindung des stromaufwärtigen Primers und eines stromabwärtigen Primers zu ermöglichen;
wobei:
(i) die amplifizierte Zielnukleinsäuresequenz SEQ ID NO: 1 umfasst, und der stromaufwärtige Primer SEQ ID NO: 2 oder eine Variante davon umfasst und der stromabwärtige Primer SEQ ID NO: 3 oder eine Variante davon umfasst und das Stranginvasionsoligonukleotid SEQ ID NO: 4 oder eine Variante davon umfasst;
(ii) die amplifizierte Zielnukleinsäuresequenz SEQ ID NO: 6 umfasst, und der stromaufwärtige Primer SEQ ID NO: 7 oder eine Variante davon umfasst und der stromabwärtige Primer SEQ ID NO: 8 oder eine Variante davon umfasst und das Stranginvasionsoligonukleotid SEQ ID NO: 9 oder eine Variante davon umfasst;
(iii) die amplifizierte Zielnukleinsäuresequenz SEQ ID NO: 11 umfasst, und der stromaufwärtige Primer SEQ ID NO: 12 oder eine Variante davon umfasst und der stromabwärtige Primer SEQ ID NO: 13 oder eine Variante davon umfasst und das Stranginvasionsoligonukleotid SEQ ID NO: 14 oder eine Variante davon umfasst;
(iv) die amplifizierte Zielnukleinsäuresequenz SEQ ID NO: 16 umfasst, und der stromaufwärtige Primer SEQ ID NO: 17 oder eine Variante davon umfasst und der stromabwärtige Primer SEQ ID NO: 18 oder eine Variante davon umfasst und das Stranginvasionsoligonukleotid SEQ ID NO: 19 oder eine Variante davon umfasst;
(v) die amplifizierte Zielnukleinsäuresequenz SEQ ID NO: 21 umfasst, und der stromaufwärtige Primer SEQ ID NO: 22 oder eine Variante davon umfasst und der stromabwärtige Primer SEQ ID NO: 23 oder eine Variante davon umfasst und das Stranginvasionsoligonukleotid SEQ ID NO: 24 oder eine Variante davon umfasst;
(vi) die amplifizierte Zielnukleinsäuresequenz SEQ ID NO: 26 umfasst, und der stromaufwärtige Primer SEQ ID NO: 27 oder eine Variante davon umfasst und der stromabwärtige Primer SEQ ID NO: 28 oder eine Variante davon umfasst und das Stranginvasionsoligonukleotid SEQ ID NO: 29 oder eine Variante davon umfasst;
(vii) die amplifizierte Zielnukleinsäuresequenz SEQ ID NO: 31 umfasst, und der stromaufwärtige Primer SEQ ID NO: 32 oder eine Variante davon umfasst und der stromabwärtige Primer SEQ ID NO: 33 oder eine Variante davon umfasst und das Stranginvasionsoligonukleotid SEQ ID NO: 34 oder eine Variante davon umfasst;
(viii) die amplifizierte Zielnukleinsäuresequenz SEQ ID NO: 36 umfasst, und der stromaufwärtige Primer SEQ ID NO: 37 oder eine Variante davon umfasst und der stromabwärtige Primer SEQ ID NO: 38 oder eine Variante davon umfasst und das Stranginvasionsoligonukleotid SEQ ID NO: 39 oder eine Variante davon umfasst;
(ix) die amplifizierte Zielnukleinsäuresequenz SEQ ID NO: 41 umfasst, und der stromaufwärtige Primer SEQ ID NO: 42 oder eine Variante davon umfasst und der stromabwärtige Primer SEQ ID NO: 43 oder eine Variante davon umfasst und das Stranginvasionsoligonukleotid SEQ ID NO: 44 oder eine Variante davon umfasst; oder
(x) die amplifizierte Zielnukleinsäuresequenz SEQ ID NO: 46 umfasst, und der stromaufwärtige Primer SEQ ID NO: 47 oder eine Variante davon umfasst und der stromabwärtige Primer SEQ ID NO: 48 oder eine Variante davon umfasst und das Stranginvasionsoligonukleotid SEQ ID NO: 49 oder eine Variante davon umfasst;
ferner wobei jeder der Primer ein Oligonukleotid mit einer Länge von weniger als 30 Nukleotiden ist und wobei jedes Stranginvasionsoligonukleotid ein Oligonukleotid mit einer Länge von mehr als 30 Nukleotiden ist und wobei eine der Variante eines Primers mindestens 70 % Sequenzidentität der Sequenz der entsprechenden ursprünglichen Sequenz aufweist, und wobei
eine der Variante eines Stranginvasionsoligonukleotids eine Region umfasst, die zu der Zielnukleinsäuresequenz komplementär ist, die mindestens 70 % Sequenzidentität der Region aufweist, die zu der Zielnukleinsäuresequenz des ursprünglichen Stranginvasionsoligonukleotids komplementär ist, oder eine komplementäre Region zu der Region umfasst, die zu der Zielnukleinsäuresequenz des ursprünglichen Stranginvasionsoligonukleotids komplementär ist.

2. Verfahren nach Anspruch 1, wobei das Stranginvasionsoligonukleotid ein oder mehrere modifizierte Nukleotide in seiner 3'-Region umfasst.

3. Verfahren nach Anspruch 1 oder 2, wobei die Variante der Primersequenzen eine Sequenz mit 1, 2, 3 oder 4 Fehlpaarungen zur Sequenz der ursprünglichen Primersequenz umfasst und die Variante des Stranginvasionsoligonukleotids eine Region umfasst, die zu der Zielnukleinsäuresequenz komplementär ist, die 1, 2, 3 oder 4 Fehlpaarungen in Bezug auf die entsprechende Region in dem ursprünglichen Stranginvasionsoligonukleotid aufweist.

4. Verfahren nach Anspruch 1 oder 2, wobei:
(i) die amplifizierte Zielnukleinsäuresequenz SEQ ID NO: 1 umfasst und das Stranginvasionsoligonukleotid SEQ ID NO: 5 ist;
(ii) die amplifizierte Zielnukleinsäuresequenz SEQ ID NO: 6 umfasst und das Stranginvasionsoligonukleotid SEQ ID NO: 10 ist;
(iii) die amplifizierte Zielnukleinsäuresequenz SEQ ID NO: 11 umfasst und das Stranginvasionsoligonukleotid SEQ ID NO: 15 ist;
(iv) die amplifizierte Zielnukleinsäuresequenz SEQ ID NO: 16 umfasst und das Stranginvasionsoligonukleotid SEQ ID NO: 20 ist;
(v) die amplifizierte Zielnukleinsäuresequenz SEQ ID NO: 21 umfasst und das Stranginvasionsoligonukleotid SEQ ID NO: 25 ist;
(vi) die amplifizierte Zielnukleinsäuresequenz SEQ ID NO: 26 umfasst und das Stranginvasionsoligonukleotid SEQ ID NO: 30 ist;
(vii) die amplifizierte Zielnukleinsäuresequenz SEQ ID NO: 31 und das Stranginvasionsoligonukleotid SEQ ID NO: 35 ist;
(viii) die amplifizierte Zielnukleinsäuresequenz SEQ ID NO: 36 und das Stranginvasionsoligonukleotid SEQ ID NO: 40 ist;
(ix) die amplifizierte Zielnukleinsäuresequenz SEQ ID NO: 41 umfasst und das Stranginvasionsoligonukleotid SEQ ID NO: 45 ist;
(x) die amplifizierte Zielnukleinsäuresequenz SEQ ID NO: 46 umfasst und das Stranginvasionsoligonukleotid SEQ ID NO: 50 ist;

5. Verfahren nach einem der vorstehenden Ansprüche, das ferner Inkontaktbringen der Probe mit einer Rekombinase umfasst.

6. Verfahren nach einem der vorhergehenden Ansprüche, wobei das Stranginvasionsoligonukleotid eine 5'-terminale Region aufweist, die zu der Zielnukleinsäuresequenz nicht komplementär ist, die eine Länge von mindestens ungefähr 14 Nukleotiden hat und/oder Purinnukleotide umfasst, die vorzugsweise im Wesentlichen aus Cytosinnukleotiden bestehen.

7. Zusammensetzung, umfassend (a) einen stromaufwärtigen Primer, (b) einen stromabwärtigen Primer und (c) ein Stranginvasionsoligonukleotid,
wobei jedes Oligonukleotid wie in einem der Ansprüche 1(i) bis 1(x) definiert ist.

8. Kit umfassend (a) einen stromaufwärtigen Primer, (b) einen stromabwärtigen Primer und (c) ein Stranginvasionsoligonukleotid,
wobei jedes Oligonukleotid wie in einem der Ansprüche 1(i) bis 1(x) definiert ist.

9. Zusammensetzung oder Kit nach Anspruch 7 oder 8, ferner umfassend
(i) eine reverse Transkriptase; und/oder
(ii) eine DNA-Polymerase; und/oder
(iii) eine Rekombinase.

10. Verfahren zur Diagnose einer Infektion durch ein Pathogen in einem Subjekt, umfassend Ausführen eines Verfahrens nach einem der Ansprüche 1 bis 6 in einer Probe von dem Subjekt.

## Revendications

1. Procédé de détection d'un acide ribonucléique (RNA) comprenant une séquence d'acide nucléique cible d'un virus de la grippe dans un échantillon, ledit procédé comprenant la mise en contact dudit échantillon avec une transcriptase inverse, au moins une amorce en amont, au moins une amorce en aval et au moins un oligonucléotide d'invasion de brin dans des conditions favorisant l'amplification de ladite séquence d'acide nucléique cible,
dans lequel chaque amorce et ledit oligonucléotide d'invasion de brin comprennent une région complémentaire de ladite séquence d'acide nucléique cible;
dans lequel ledit oligonucléotide d'invasion de brin rend au moins une partie d'un acide nucléique duplex comprenant ladite séquence d'acide nucléique cible en simple brin, pour permettre la liaison de ladite amorce en amont et d'une amorce en aval ; dans lequel :
(i) la séquence d'acide nucléique cible amplifiée comprend SEQ ID NO: 1 et l'amorce en amont comprend SEQ ID NO: 2 ou un variant de celle-ci et l'amorce en aval comprend SEQ ID NO: 3 ou un variant de celle-ci et l'oligonucléotide d'invasion de brin comprend SEQ ID NO: 4 ou un variant de celle-ci ;
(ii) la séquence d'acide nucléique cible amplifiée comprend SEQ ID NO: 6 et l'amorce en amont comprend SEQ ID NO: 7 ou un variant de celle-ci et l'amorce en aval comprend SEQ ID NO: 8 ou un variant de celle-ci et l'oligonucléotide d'invasion de brin comprend SEQ ID NO: 9 ou un variant de celle-ci ;
(iii) la séquence d'acide nucléique cible amplifiée comprend SEQ ID NO: 11 et l'amorce en amont comprend SEQ ID NO: 12 ou un variant de celle-ci et l'amorce en aval comprend SEQ ID NO: 13 ou un variant de celle-ci et l'oligonucléotide d'invasion de brin comprend SEQ ID NO: 14 ou un variant de celle-ci ;
(iv) la séquence d'acide nucléique cible amplifiée comprend SEQ ID NO: 16 et l'amorce en amont comprend SEQ ID NO: 17 ou un variant de celle-ci et l'amorce en aval comprend SEQ ID NO: 18 ou un variant de celle-ci et l'oligonucléotide d'invasion de brin comprend SEQ ID NO: 19 ou un variant de celle-ci ;
(v) la séquence d'acide nucléique cible amplifiée comprend SEQ ID NO: 21 et l'amorce en amont comprend SEQ ID NO: 22 ou un variant de celle-ci et l'amorce en aval comprend SEQ ID NO: 23 ou un variant de celle-ci et l'oligonucléotide d'invasion de brin comprend SEQ ID NO: 24 ou un variant de celle-ci ;
(vi) la séquence d'acide nucléique cible amplifiée comprend SEQ ID NO: 26 et l'amorce en amont comprend SEQ ID NO: 27 ou un variant de celle-ci et l'amorce en aval comprend SEQ ID NO: 28 ou un variant de celle-ci et l'oligonucléotide d'invasion de brin comprend SEQ ID NO: 29 ou un variant de celle-ci ;
(vii) la séquence d'acide nucléique cible amplifiée comprend SEQ ID NO: 31 et l'amorce en amont comprend SEQ ID NO: 32 ou un variant de celle-ci et l'amorce en aval comprend SEQ ID NO: 33 ou un variant de celle-ci et l'oligonucléotide d'invasion de brin comprend SEQ ID NO: 34 ou un variant de celle-ci ;
(viii) la séquence d'acide nucléique cible amplifiée comprend SEQ ID NO: 36 et l'amorce en amont comprend SEQ ID NO: 37 ou un variant de celle-ci et l'amorce en aval comprend SEQ ID NO: 38 ou un variant de celle-ci et l'oligonucléotide d'invasion de brin comprend SEQ ID NO: 39 ou un variant de celle-ci ;
(ix) la séquence d'acide nucléique cible amplifiée comprend SEQ ID NO: 41 et l'amorce en amont comprend SEQ ID NO: 42 ou un variant de celle-ci et l'amorce en aval comprend SEQ ID NO: 43 ou un variant de celle-ci et l'oligonucléotide d'invasion de brin comprend SEQ ID NO: 44 ou un variant de celle-ci ; ou
(x) la séquence d'acide nucléique cible amplifiée comprend SEQ ID NO: 46 et l'amorce en amont comprend SEQ ID NO: 47 ou un variant de celle-ci et l'amorce en aval comprend SEQ ID NO: 48 ou un variant de celle-ci et l'oligonucléotide d'invasion de brin comprend SEQ ID NO: 49 ou un variant de celle-ci ;
dans lequel chaque amorce est en outre un oligonucléotide de moins de 30 nucléotides de longueur et dans lequel chaque oligonucléotide d'invasion de brin est un oligonucléotide de plus de 30 nucléotides de longueur, et dans lequel ledit variant d'une amorce a au moins 70% d'identité de séquence avec la séquence de la séquence d'origine correspondante, et dans lequel un
dit variant d'un oligonucléotide d'invasion de brin comprend une région complémentaire de ladite séquence d'acide nucléique cible ayant au moins 70% d'identité de séquence avec la région complémentaire de ladite séquence d'acide nucléique cible de l'oligonucléotide d'invasion de brin d'origine, ou comprend une région complémentaire à la région complémentaire de ladite séquence d'acide nucléique cible de l'oligonucléotide d'invasion de brin d'origine.

2. Procédé selon la revendication 1, dans lequel l'oligonucléotide d'invasion de brin comprend un ou plusieurs nucléotides modifiés dans sa région 3'.

3. Procédé selon la revendication 1 ou 2, dans lequel le variant des séquences d'amorces comprend une séquence comportant 1, 2, 3 ou 4 mésappariements avec la séquence de la séquence d'amorces d'origine et le variant de l'oligonucléotide d'invasion de brin comprend une région complémentaire de la séquence d'acide nucléique cible ayant 1, 2, 3 ou 4 mésappariements par rapport à la région correspondante dans la séquence de l'oligonucléotide d'invasion de brin.

4. Procédé selon la revendication 1 ou 2, dans lequel :
(i) la séquence d'acide nucléique cible amplifiée comprend SEQ ID NO: 1 et l'oligonucléotide d'invasion de brin est SEQ ID NO: 5 ;
(ii) la séquence d'acide nucléique cible amplifiée comprend SEQ ID NO: 6 et l'oligonucléotide d'invasion de brin est SEQ ID NO: 10 ;
(iii) la séquence d'acide nucléique cible amplifiée comprend SEQ ID NO: 11 et l'oligonucléotide d'invasion de brin est SEQ ID NO: 15 ;
(iv) la séquence d'acide nucléique cible amplifiée comprend SEQ ID NO: 16 et l'oligonucléotide d'invasion de brin est SEQ ID NO: 20 ;
(v) la séquence d'acide nucléique cible amplifiée comprend SEQ ID NO: 21 et l'oligonucléotide d'invasion de brin est SEQ ID NO: 25 ;
(vi) la séquence d'acide nucléique cible amplifiée comprend SEQ ID NO: 26 et l'oligonucléotide d'invasion de brin est SEQ ID NO: 30 ;
(vii) la séquence d'acide nucléique cible amplifiée comprend SEQ ID NO: 31 et l'oligonucléotide d'invasion de brin est SEQ ID NO: 35 ;
(viii) la séquence d'acide nucléique cible amplifiée comprend SEQ ID NO: 36 et l'oligonucléotide d'invasion de brin est SEQ ID NO: 40 ;
(ix) la séquence d'acide nucléique cible amplifiée comprend SEQ ID NO: 41 et l'oligonucléotide d'invasion de brin est SEQ ID NO: 45 ;
(x) la séquence d'acide nucléique cible amplifiée comprend SEQ ID NO: 46 et l'oligonucléotide d'invasion de brin est SEQ ID NO: 50 ;

5. Procédé selon l'une quelconque des revendications précédentes, qui comprend en outre la mise en contact dudit échantillon avec une recombinase.

6. Procédé selon l'une quelconque des revendications précédentes, dans lequel l'oligonucléotide d'invasion de brin a une région terminale 5' non complémentaire de la séquence d'acide nucléique cible qui a une longueur d'au moins environ 14 nucléotides et / ou est composée de nucléotides de purine constituée de préférence essentiellement de nucléotides cytosine.

7. Composition comprenant (a) une amorce en amont, (b) une amorce en aval et (c) un oligonucléotide d'invasion de brin,
dans laquelle chaque oligonucléotide est tel que défini dans l'une quelconque des revendications 1 (i) à 1 (x).

8. Trousse comprenant (a) une amorce en amont, (b) une amorce en aval et (c) un oligonucléotide d'invasion de brin,
dans laquelle chaque oligonucléotide est tel que défini dans l'une quelconque des revendications 1 (i) à 1 (x).

9. Composition ou trousse selon la revendication 7 ou 8, comprenant en outre
(i) une transcriptase inverse ; et / ou
(ii) une ADN polymérase ; et / ou
(iii) une recombinase.

10. Procédé de diagnostic d'une infection par un agent pathogène chez un sujet, comprenant la mise en oeuvre d'un procédé tel que défini dans l'une quelconque des revendications 1 à 6, dans un échantillon dudit sujet.


### SEQUENCE LISTING

<110> ORION DIAGNOSTICA OY
<120> METHOD
<130> N407015WO
<140> tbc
   <141> 2016-12-08
<150> EP15199650.1
   <151> 2015-12-11
<160> 61
<170> PatentIn version 3.5
<210> 1
   <211> 66
   <212> DNA
   <213> Artificial Sequence
<220>
   <223> amplicon for IFAX
<400> 1
   aattgatggc catccgaatt cttttggtcg ctgtctggct gtcagtaagt atgctagagt 60
   cccgtt 66
<210> 2
   <211> 20
   <212> DNA
   <213> Artificial Sequence
<220>
   <223> forward primer for SEQ ID NO: 1
<400> 2
   aattgatggc catccgaatt 20
<210> 3
   <211> 16
   <212> DNA
   <213> Artificial Sequence
<220>
   <223> reverse primer for SEQ ID NO:1
<400> 3
   aacgggactc tagcat 16
<210> 4
   <211> 53
   <212> DNA
   <213> Artificial Sequence
<220>
   <223> DNA equivalent of SEQ ID NO:5
<400> 4
   cccccccccc ccccgaattc ttttggtcgc tgtctggctg tcagtaagta tgc 53
<210> 5
   <211> 53
   <212> DNA
   <213> Artificial Sequence
<220>
   <223> specific invasion oligo used for SEQ ID NO:1
<220>
   <221> misc_feature
   <222> (44)..(44)
   <223> 2'-O-METHYL RNA
<220>
   <221> misc_feature
   <222> (45)..(45)
   <223> 2'-O-METHYL RNA
<220>
   <221> misc_feature
   <222> (46)..(46)
   <223> 2'-O-METHYL RNA
<220>
   <221> misc_feature
   <222> (47)..(47)
   <223> 2'-O-METHYL RNA
<220>
   <221> misc_feature
   <222> (48)..(48)
   <223> 2'-O-METHYL RNA
<220>
   <221> misc_feature
   <222> (49)..(49)
   <223> 2'-O-METHYL RNA
<220>
   <221> misc_feature
   <222> (50)..(50)
   <223> 2'-O-METHYL RNA
<220>
   <221> misc_feature
   <222> (51)..(51)
   <223> 2'-O-METHYL RNA
<220>
   <221> misc_feature
   <222> (52)..(52)
   <223> 2'-O-METHYL RNA
<220>
   <221> misc_feature
   <222> (53)..(53)
   <223> 2'-O-METHYL RNA
<400> 5
   cccccccccc ccccgaattc ttttggtcgc tgtctggctg tcaguaagua ugc 53
<210> 6
   <211> 69
   <212> DNA
   <213> Artificial Sequence
<220>
   <223> amplicon for IFB4
<400> 6
   actacaataa tacaaaaggc caaaaacaca atggcagaat ttagtgaaga tcctgaatta 60
   caaccagca 69
<210> 7
   <211> 18
   <212> DNA
   <213> Artificial Sequence
<220>
   <223> forward primer for SEQ ID NO: 6
<400> 7
   actacaataa tacaaaag 18
<210> 8
   <211> 18
   <212> DNA
   <213> Artificial Sequence
<220>
   <223> reverse primer for SEQ ID NO:6
<400> 8
   tgctggttgt aattcagg 18
<210> 9
   <211> 55
   <212> DNA
   <213> Artificial Sequence
<220>
   <223> DNA equivalent of SEQ ID NO:10
<400> 9
   cccccccccc ccccaaggcc aaaaacacaa tggcagaatt tagtgaagat cctga 55
<210> 10
   <211> 55
   <212> DNA
   <213> Artificial Sequence
<220>
   <223> specific invasion oligo used for SEQ ID NO:6
<220>
   <221> misc_feature
   <222> (43)..(43)
   <223> 2'-O-METHYL RNA
<220>
   <221> misc_feature
   <222> (44)..(44)
   <223> 2'-O-METHYL RNA
<220>
   <221> misc_feature
   <222> (45)..(45)
   <223> 2'-O-METHYL RNA
<220>
   <221> misc_feature
   <222> (46)..(46)
   <223> 2'-O-METHYL RNA
<220>
   <221> misc_feature
   <222> (47)..(47)
   <223> 2'-O-METHYL RNA
<220>
   <221> misc_feature
   <222> (48)..(48)
   <223> 2'-O-METHYL RNA
<220>
   <221> misc_feature
   <222> (49)..(49)
   <223> 2'-O-METHYL RNA
<220>
   <221> misc_feature
   <222> (50)..(50)
   <223> 2'-O-METHYL RNA
<220>
   <221> misc_feature
   <222> (51)..(51)
   <223> 2'-O-METHYL RNA
<220>
   <221> misc_feature
   <222> (52)..(52)
   <223> 2'-O-METHYL RNA
<220>
   <221> misc_feature
   <222> (53)..(53)
   <223> 2'-O-METHYL RNA
<220>
   <221> misc_feature
   <222> (54)..(54)
   <223> 2'-O-METHYL RNA
<220>
   <221> misc_feature
   <222> (55)..(55)
   <223> 2'-O-METHYL RNA
<400> 10
   cccccccccc ccccaaggcc aaaaacacaa tggcagaatt tagugaagau ccuga 55
<210> 11
   <211> 69
   <212> DNA
   <213> Artificial Sequence
<220>
   <223> amplicon for IFA1
<400> 11
   acgaaaacgg gactctagca tacttactga cagccagaca gcgaccaaaa gaattcggat 60
   ggccatcaa 69
<210> 12
   <211> 20
   <212> DNA
   <213> Artificial Sequence
<220>
   <223> forward primer for SEQ ID NO: 11
<400> 12
   acgaaaacgg gactctagca 20
<210> 13
   <211> 18
   <212> DNA
   <213> Artificial Sequence
<220>
   <223> reverse primer for SEQ ID NO:11
<400> 13
   ttgatggcca tccgaatt 18
<210> 14
   <211> 56
   <212> DNA
   <213> Artificial Sequence
<220>
   <223> DNA equivalent of SEQ ID NO:15
<220>
   <221> misc_feature
   <222> (56)..(56)
   <223> mismatch
<400> 14
   cccccccccc ccccctagca tacttactga cagccagaca gcgaccaaaa gaatta 56
<210> 15
   <211> 56
   <212> DNA
   <213> Artificial Sequence
<220>
   <223> specific invasion oligo used for SEQ ID NO:11
<220>
   <221> misc_feature
   <222> (43)..(43)
   <223> 2'-O-METHYL RNA
<220>
   <221> misc_feature
   <222> (44)..(44)
   <223> 2'-O-METHYL RNA
<220>
   <221> misc_feature
   <222> (45)..(45)
   <223> 2'-O-METHYL RNA
<220>
   <221> misc_feature
   <222> (46)..(46)
   <223> 2'-O-METHYL RNA
<220>
   <221> misc_feature
   <222> (47)..(47)
   <223> 2'-O-METHYL RNA
<220>
   <221> misc_feature
   <222> (48)..(48)
   <223> 2'-O-METHYL RNA
<220>
   <221> misc_feature
   <222> (49)..(49)
   <223> 2'-O-METHYL RNA
<220>
   <221> misc_feature
   <222> (50)..(50)
   <223> 2'-O-METHYL RNA
<220>
   <221> misc_feature
   <222> (51)..(51)
   <223> 2'-O-METHYL RNA
<220>
   <221> misc_feature
   <222> (52)..(52)
   <223> 2'-O-METHYL RNA
<220>
   <221> misc_feature
   <222> (53)..(53)
   <223> 2'-O-METHYL RNA
<220>
   <221> misc_feature
   <222> (55)..(55)
   <223> 2'-O-METHYL RNA
<220>
   <221> misc_feature
   <222> (56)..(56)
   <223> 2'-O-METHYL RNA
<220>
   <221> misc_feature
   <222> (56)..(56)
   <223> mismatch
<400> 15
   cccccccccc ccccctagca tacttactga cagccagaca gcgaccaaaa gaauua 56
<210> 16
   <211> 65
   <212> DNA
   <213> Artificial Sequence
<220>
   <223> amplicon for INFLA3
<400> 16
   ttgaaccaag acgcattgag caaaacccag ggatcattaa tcaggcactc ctcaattgct 60
   tcata 65
<210> 17
   <211> 21
   <212> DNA
   <213> Artificial Sequence
<220>
   <223> forward primer for SEQ ID NO: 16
<400> 17
   ttgaaccaag acgcattgag c 21
<210> 18
   <211> 15
   <212> DNA
   <213> Artificial Sequence
<220>
   <223> reverse primer for SEQ ID NO:16
<400> 18
   tatgaagcaa ttgag 15
<210> 19
   <211> 55
   <212> DNA
   <213> Artificial Sequence
<220>
   <223> DNA equivalent of SEQ ID NO:20
<400> 19
   ttctccttac actccattga gcaaaaccca gggatcatta atcaggcact cctca 55
<210> 20
   <211> 55
   <212> DNA
   <213> Artificial Sequence
<220>
   <223> specific invasion oligo used for SEQ ID NO:16
<220>
   <221> misc_feature
   <222> (43)..(43)
   <223> 2'-O-METHYL RNA
<220>
   <221> misc_feature
   <222> (45)..(45)
   <223> 2'-O-METHYL RNA
<220>
   <221> misc_feature
   <222> (47)..(47)
   <223> 2'-O-METHYL RNA
<220>
   <221> misc_feature
   <222> (49)..(49)
   <223> 2'-O-METHYL RNA
<220>
   <221> misc_feature
   <222> (51)..(51)
   <223> 2'-O-METHYL RNA
<220>
   <221> misc_feature
   <222> (53)..(53)
   <223> 2'-O-METHYL RNA
<220>
   <221> misc_feature
   <222> (55)..(55)
   <223> 2'-O-METHYL RNA
<400> 20
   ttctccttac actccattga gcaaaaccca gggatcatta atcaggcact ccuca 55
<210> 21
   <211> 66
   <212> DNA
   <213> Artificial Sequence
<220>
   <223> amplicon for IFA2
<400> 21
   gttcacgctc accgtgccca gtgagcgagg actgcagcgt agacgctttg tccaaaatgc 60
   cctaaa 66
<210> 22
   <211> 18
   <212> DNA
   <213> Artificial Sequence
<220>
   <223> forward primer for SEQ ID NO: 21
<400> 22
   gttcacgctc accgtgcc 18
<210> 23
   <211> 16
   <212> DNA
   <213> Artificial Sequence
<220>
   <223> reverse primer for SEQ ID NO:21
<400> 23
   tttagggcat tttgga 16
<210> 24
   <211> 56
   <212> DNA
   <213> Artificial Sequence
<220>
   <223> DNA equivalent of SEQ ID NO:25
<220>
   <221> misc_feature
   <222> (14)..(14)
   <223> T in seeding
<400> 24
   cccccccccc ccctcgtgcc cagtgagcga ggactgcagc gtagacgctt tgtcca 56
<210> 25
   <211> 56
   <212> DNA
   <213> Artificial Sequence
<220>
   <223> specific invasion oligo used for SEQ ID NO:25
<220>
   <221> misc_feature
   <222> (14)..(14)
   <223> T in seeding
<220>
   <221> misc_feature
   <222> (43)..(43)
   <223> 2'-O-METHYL RNA
<220>
   <221> misc_feature
   <222> (44)..(44)
   <223> 2'-O-METHYL RNA
<220>
   <221> misc_feature
   <222> (45)..(45)
   <223> 2'-O-METHYL RNA
<220>
   <221> misc_feature
   <222> (46)..(46)
   <223> 2'-O-METHYL RNA
<220>
   <221> misc_feature
   <222> (47)..(47)
   <223> 2'-O-METHYL RNA
<220>
   <221> misc_feature
   <222> (48)..(48)
   <223> 2'-O-METHYL RNA
<220>
   <221> misc_feature
   <222> (49)..(49)
   <223> 2'-O-METHYL RNA
<220>
   <221> misc_feature
   <222> (50)..(50)
   <223> 2'-O-METHYL RNA
<220>
   <221> misc_feature
   <222> (51)..(51)
   <223> 2'-O-METHYL RNA
<220>
   <221> misc_feature
   <222> (52)..(52)
   <223> 2'-O-METHYL RNA
<220>
   <221> misc_feature
   <222> (53)..(53)
   <223> 2'-O-METHYL RNA
<220>
   <221> misc_feature
   <222> (54)..(54)
   <223> 2'-O-METHYL RNA
<220>
   <221> misc_feature
   <222> (55)..(55)
   <223> 2'-O-METHYL RNA
<220>
   <221> misc_feature
   <222> (56)..(56)
   <223> 2'-O-METHYL RNA
<400> 25
   cccccccccc ccctcgtgcc cagtgagcga ggactgcagc gtagacgcuu ugucca 56
<210> 26
   <211> 69
   <212> DNA
   <213> Artificial Sequence
<220>
   <223> amplicon for IFA4
<400> 26
   ggtaatgaaa cgaaaacggg actctagtat acttactgac agccagacag cgaccaaaag 60
   aattcggat 69
<210> 27
   <211> 16
   <212> DNA
   <213> Artificial Sequence
<220>
   <223> forward primer for SEQ ID NO: 26
<400> 27
   ggtaatgaaa cgaaaa 16
<210> 28
   <211> 17
   <212> DNA
   <213> Artificial Sequence
<220>
   <223> reverse primer for SEQ ID NO:26
<400> 28
   atccgaattc ttttggt 17
<210> 29
   <211> 58
   <212> DNA
   <213> Artificial Sequence
<220>
   <223> DNA equivalent of SEQ ID NO:30
<400> 29
   cccccccccc ccccaaacgg gactctagta tacttactga cagccagaca gcgaccaa 58
<210> 30
   <211> 58
   <212> DNA
   <213> Artificial Sequence
<220>
   <223> specific IO used for SEQ ID NO:26
<220>
   <221> misc_feature
   <222> (42)..(42)
   <223> 2'-O-METHYL RNA
<220>
   <221> misc_feature
   <222> (43)..(43)
   <223> 2'-O-METHYL RNA
<220>
   <221> misc_feature
   <222> (44)..(44)
   <223> 2'-O-METHYL RNA
<220>
   <221> misc_feature
   <222> (45)..(45)
   <223> 2'-O-METHYL RNA
<220>
   <221> misc_feature
   <222> (46)..(46)
   <223> 2'-O-METHYL RNA
<220>
   <221> misc_feature
   <222> (47)..(47)
   <223> 2'-O-METHYL RNA
<220>
   <221> misc_feature
   <222> (48)..(48)
   <223> 2'-O-METHYL RNA
<220>
   <221> misc_feature
   <222> (49)..(49)
   <223> 2'-O-METHYL RNA
<220>
   <221> misc_feature
   <222> (50)..(50)
   <223> 2'-O-METHYL RNA
<220>
   <221> misc_feature
   <222> (51)..(51)
   <223> 2'-O-METHYL RNA
<220>
   <221> misc_feature
   <222> (52)..(52)
   <223> 2'-O-METHYL RNA
<220>
   <221> misc_feature
   <222> (53)..(53)
   <223> 2'-O-METHYL RNA
<220>
   <221> misc_feature
   <222> (54)..(54)
   <223> 2'-O-METHYL RNA
<220>
   <221> misc_feature
   <222> (55)..(55)
   <223> 2'-O-METHYL RNA
<220>
   <221> misc_feature
   <222> (56)..(56)
   <223> 2'-O-METHYL RNA
<220>
   <221> misc_feature
   <222> (57)..(57)
   <223> 2'-O-METHYL RNA
<220>
   <221> misc_feature
   <222> (58)..(58)
   <223> 2'-O-METHYL RNA
<400> 30
   cccccccccc ccccaaacgg gactctagta tacttactga cagccagaca gcgaccaa 58
<210> 31
   <211> 67
   <212> DNA
   <213> Artificial Sequence
<220>
   <223> amplicion for IFA5
<400> 31
   aatgaaacga aaacgggact ctagtatact tactgacagc cagacagcga ccaaaagaat 60
   tcggatg 67
<210> 32
   <211> 18
   <212> DNA
   <213> Artificial Sequence
<220>
   <223> forward primer for SEQ ID NO: 31
<400> 32
   aatgaaacga aaacggga 18
<210> 33
   <211> 15
   <212> DNA
   <213> Artificial Sequence
<220>
   <223> reverse primer for SEQ ID NO:31
<400> 33
   catccgaatt ctttt 15
<210> 34
   <211> 55
   <212> DNA
   <213> Artificial Sequence
<220>
   <223> DNA equivalent of SEQ ID NO:35
<400> 34
   cccccccccc ccccggactc tagtatactt actgacagcc agacagcgac caaaa 55
<210> 35
   <211> 55
   <212> DNA
   <213> Artificial Sequence
<220>
   <223> specific invasion oligo used for SEQ ID NO:31
<220>
   <221> misc_feature
   <222> (43)..(43)
   <223> 2'-O-METHYL RNA
<220>
   <221> misc_feature
   <222> (44)..(44)
   <223> 2'-O-METHYL RNA
<220>
   <221> misc_feature
   <222> (45)..(45)
   <223> 2'-O-METHYL RNA
<220>
   <221> misc_feature
   <222> (46)..(46)
   <223> 2'-O-METHYL RNA
<220>
   <221> misc_feature
   <222> (47)..(47)
   <223> 2'-O-METHYL RNA
<220>
   <221> misc_feature
   <222> (48)..(48)
   <223> 2'-O-METHYL RNA
<220>
   <221> misc_feature
   <222> (49)..(49)
   <223> 2'-O-METHYL RNA
<220>
   <221> misc_feature
   <222> (50)..(50)
   <223> 2'-O-METHYL RNA
<220>
   <221> misc_feature
   <222> (51)..(51)
   <223> 2'-O-METHYL RNA
<220>
   <221> misc_feature
   <222> (52)..(52)
   <223> 2'-O-METHYL RNA
<220>
   <221> misc_feature
   <222> (53)..(53)
   <223> 2'-O-METHYL RNA
<220>
   <221> misc_feature
   <222> (54)..(54)
   <223> 2'-O-METHYL RNA
<220>
   <221> misc_feature
   <222> (55)..(55)
   <223> 2'-O-METHYL RNA
<400> 35
   cccccccccc ccccggactc tagtatactt actgacagcc agacagcgac caaaa 55
<210> 36
   <211> 69
   <212> DNA
   <213> Artificial Sequence
<220>
   <223> amplicon for IFAbl
<400> 36
   gcgacaatgc ttcaacccga tgattgtcga acttgcagaa aaagcaatga aagagtatgg 60
   ggaggatct 69
<210> 37
   <211> 17
   <212> DNA
   <213> Artificial Sequence
<220>
   <223> forward primer for SEQ ID NO:36
<400> 37
   gcgacaatgc ttcaacc 17
<210> 38
   <211> 17
   <212> DNA
   <213> Artificial Sequence
<220>
   <223> reverse primer for SEQ ID NO:36
<400> 38
   agatcctccc catactc 17
<210> 39
   <211> 55
   <212> DNA
   <213> Artificial Sequence
<220>
   <223> DNA equivalent of SEQ ID NO:40
<400> 39
   ttctccttac actcacccga tgattgtcga acttgcagaa aaagcaatga aagag 55
<210> 40
   <211> 55
   <212> DNA
   <213> Artificial Sequence
<220>
   <223> specific invasion oligo used for SEQ ID NO:36
<220>
   <221> misc_feature
   <222> (44)..(44)
   <223> 2'-O-METHYL RNA
<220>
   <221> misc_feature
   <222> (45)..(45)
   <223> 2'-O-METHYL RNA
<220>
   <221> misc_feature
   <222> (46)..(46)
   <223> 2'-O-METHYL RNA
<220>
   <221> misc_feature
   <222> (47)..(47)
   <223> 2'-O-METHYL RNA
<220>
   <221> misc_feature
   <222> (48)..(48)
   <223> 2'-O-METHYL RNA
<220>
   <221> misc_feature
   <222> (49)..(49)
   <223> 2'-O-METHYL RNA
<220>
   <221> misc_feature
   <222> (50)..(50)
   <223> 2'-O-METHYL RNA
<220>
   <221> misc_feature
   <222> (51)..(51)
   <223> 2'-O-METHYL RNA
<220>
   <221> misc_feature
   <222> (52)..(52)
   <223> 2'-O-METHYL RNA
<220>
   <221> misc_feature
   <222> (53)..(53)
   <223> 2'-O-METHYL RNA
<220>
   <221> misc_feature
   <222> (54)..(54)
   <223> 2'-O-METHYL RNA
<220>
   <221> misc_feature
   <222> (55)..(55)
   <223> 2'-O-METHYL RNA
<400> 40
   ttctccttac actcacccga tgattgtcga acttgcagaa aaagcaauga aagag 55
<210> 41
   <211> 68
   <212> DNA
   <213> Artificial Sequence
<220>
   <223> amplicon for IFAbl4
<400> 41
   accataagac aagagatggc caacagaggc ctctgggatt cctttcgtca gtccgaaaga 60
   ggcgaaga 68
<210> 42
   <211> 18
   <212> DNA
   <213> Artificial Sequence
<220>
   <223> forward primer for SEQ ID NO:41
<400> 42
   accataagac aagagatg 18
<210> 43
   <211> 18
   <212> DNA
   <213> Artificial Sequence
<220>
   <223> reverse primer for SEQ ID NO:41
<400> 43
   tcttcgcctc tttcggac 18
<210> 44
   <211> 55
   <212> DNA
   <213> Artificial Sequence
<220>
   <223> DNA equivalent of SEQ ID NO:45
<400> 44
   cccccccccc ccccatggcc aacagaggcc tctgggattc ctttcgtcag tccga 55
<210> 45
   <211> 55
   <212> DNA
   <213> Artificial Sequence
<220>
   <223> specific invasion oligo used for SEQ ID NO:41
<220>
   <221> misc_feature
   <222> (43)..(43)
   <223> 2'-O-METHYL RNA
<220>
   <221> misc_feature
   <222> (44)..(44)
   <223> 2'-O-METHYL RNA
<220>
   <221> misc_feature
   <222> (45)..(45)
   <223> 2'-O-METHYL RNA
<220>
   <221> misc_feature
   <222> (46)..(46)
   <223> 2'-O-METHYL RNA
<220>
   <221> misc_feature
   <222> (47)..(47)
   <223> 2'-O-METHYL RNA
<220>
   <221> misc_feature
   <222> (48)..(48)
   <223> 2'-O-METHYL RNA
<220>
   <221> misc_feature
   <222> (49)..(49)
   <223> 2'-O-METHYL RNA
<220>
   <221> misc_feature
   <222> (50)..(50)
   <223> 2'-O-METHYL RNA
<220>
   <221> misc_feature
   <222> (51)..(51)
   <223> 2'-O-METHYL RNA
<220>
   <221> misc_feature
   <222> (52)..(52)
   <223> 2'-O-METHYL RNA
<220>
   <221> misc_feature
   <222> (53)..(53)
   <223> 2'-O-METHYL RNA
<220>
   <221> misc_feature
   <222> (54)..(54)
   <223> 2'-O-METHYL RNA
<220>
   <221> misc_feature
   <222> (55)..(55)
   <223> 2'-O-METHYL RNA
<400> 45
   cccccccccc ccccatggcc aacagaggcc tctgggattc ctuucgucag uccga 55
<210> 46
   <211> 73
   <212> DNA
   <213> Artificial Sequence
<220>
   <223> amplicon for IFB2/3
<400> 46
   tattacaaga aacttccaga ctacaataat acaaaaggcc aaaaacacaa tggcagaatt 60
   tagtgaagat cct 73
<210> 47
   <211> 20
   <212> DNA
   <213> Artificial Sequence
<220>
   <223> forward primer for SEQ ID NO:46
<400> 47
   tattacaaga aacttccaga 20
<210> 48
   <211> 18
   <212> DNA
   <213> Artificial Sequence
<220>
   <223> reverse primer for SEQ ID NO:46
<400> 48
   aggatcttca ctaaattc 18
<210> 49
   <211> 55
   <212> DNA
   <213> Artificial Sequence
<220>
   <223> DNA equivalent of SEQ ID NO:50
<400> 49
   cccccccccc ccccagacta caataataca aaaggccaaa aacacaatgg cagaa 55
<210> 50
   <211> 55
   <212> DNA
   <213> Artificial Sequence
<220>
   <223> specific invasion oligo used for SEQ ID NO: 46
<220>
   <221> misc_feature
   <222> (43)..(43)
   <223> 2'-O-METHYL RNA
<220>
   <221> misc_feature
   <222> (45)..(45)
   <223> 2'-O-METHYL RNA
<220>
   <221> misc_feature
   <222> (47)..(47)
   <223> 2'-O-METHYL RNA
<220>
   <221> misc_feature
   <222> (49)..(49)
   <223> 2'-O-METHYL RNA
<220>
   <221> misc_feature
   <222> (51)..(51)
   <223> 2'-O-METHYL RNA
<220>
   <221> misc_feature
   <222> (53)..(53)
   <223> 2'-O-METHYL RNA
<220>
   <221> misc_feature
   <222> (55)..(55)
   <223> 2'-O-METHYL RNA
<400> 50
   cccccccccc ccccagacta caataataca aaaggccaaa aacacaatgg cagaa 55
<210> 51
   <211> 71
   <212> DNA
   <213> Influenza virus
<400> 51
   aattgatggc catccgaatt cttttggtcg ctgtctggct gtcagtaagt atgctagagt        60
   cccgttttcg t                                                               71
<210> 52
   <211> 91
   <212> DNA
   <213> Influenza virus
<400> 52
   attacaagaa acttccagac tacaataata caaaaggcca aaaacacaat ggcagaattt        60
   agtgaagatc ctgaattaca accagcaatg c                                         91
<210> 53
   <211> 70
   <212> DNA
   <213> Influenza virus
<400> 53
   acgaaaacgg gactctagca tacttactga cagccagaca gcgaccaaaa gaattcggat        60
   ggccatcaat                                                                 70
<210> 54
   <211> 75
   <212> DNA
   <213> Influenza virus
<400> 54
   ggagttgaac caagacgcat tgagcaaaac ccagggatca ttaatcaggc actcctcaat        60
   tgcttcatat agccc                                                           75
<210> 55
   <211> 71
   <212> DNA
   <213> Influenza virus
<400> 55
   tgtgttcacg ctcaccgtgc ccagtgagcg aggactgcag cgtagacgct ttgtccaaaa        60
   tgccctaaat g                                                               71
<210> 56
   <211> 88
   <212> DNA
   <213> Influenza virus
<400> 56
   ggtgttggta atgaaacgaa aacgggactc tagtatactt actgacagcc agacagcgac 60
   caaaagaatt cggatggcca tcaattaa 88
<210> 57
   <211> 79
   <212> DNA
   <213> Influenza virus
<400> 57
   ggtgttggta atgaaacgaa aacgggactc tagtatactt actgacagcc agacagcgac 60
   caaaagaatt cggatggcc 79
<210> 58
   <211> 77
   <212> DNA
   <213> Influenza virus
<400> 58
   tgtgcgacaa tgcttcaacc cgatgattgt cgaacttgca gaaaaagcaa tgaaagagta 60
   tggggaggat ctgaaaa 77
<210> 59
   <211> 73
   <212> DNA
   <213> Influenza virus
<400> 59
   tcaccataag acaagagatg gccaacagag gcctctggga ttcctttcgt cagtccgaaa 60
   gaggcgaaga aac 73
<210> 60
   <211> 92
   <212> DNA
   <213> Influenza virus
<400> 60
   tattacaaga aacttccaga ctacaataat acaaaaggcc aaaaacacaa tggcagaatt 60
   tagtgaagat cctgaattac aaccagcaat gc 92
<210> 61
   <211> 66
   <212> RNA
   <213> Artificial Sequence
<220>
   <223> RNA equivalent of SEQ ID NO:1
<400> 61
   aauugauggc cauccgaauu cuuuuggucg cugucuggcu gucaguaagu augcuagagu 60
   cccguu 66